# EUROPEAN PATENT APPLICATION

(11) **EP 4 074 705 A1**
(43) Date of publication of application: **19.10.2022**
(21) Application number: 20898545.7
(22) Date of filing: 03.12.2020
(51) Int. Cl.: C07D 405/14, C07D 409/14, C07D 471/04, C07D 487/04, C07D 495/04, H01L 51/00, H01L 51/50

(54) **HETEROCYCLIC COMPOUND, AND ORGANIC LIGHT-EMITTING ELEMENT COMPRISING SAME**

(30) Priority: 13.12.2019 KR 20190167113
(71) Applicant: LT Materials Co., Ltd., Yongin-si, Gyeonggi-do 17118 (KR)
(72) Inventor: CHOI, Eui-Jeong, Yongin-City, Gyeonggi-do 17118 (KR); NO, Young-Seok, Yongin-si, Gyeonggi-do 17118 (KR); YANG, Seung-Gyu, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2020/017525
(87) International publication number: WO 2021/118159

(57) **Abstract**

The present specification relates to a heterocyclic compound represented by Chemical Formula 1, and an organic light emitting device comprising the same.

## Description

### [Technical Field]

This application claims priority to and the benefits of Korean Patent Application No. 10-2019-0167113, filed with the Korean Intellectual Property Office on December 13, 2019, the entire contents of which are incorporated herein by reference.

The present specification relates to a heterocyclic compound, and an organic light emitting device comprising the same.

### [Background Art]

An organic electroluminescent device is one type of selfemissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a hostdopant-based light emitting layer may also be used. In addition thereto, compounds capable of performing roles of hole injection, hole transport, electron blocking, hole blocking, electron transport, electron injection and the like may also be used as a material of the organic thin film.

Development of an organic thin film material has been continuously required for enhancing performance, lifetime or efficiency of an organic light emitting device.

Studies on an organic light emitting device comprising a compound capable of satisfying conditions required for materials usable in an organic light emitting device, for example, satisfying proper energy level, electrochemical stability, thermal stability and the like, and having a chemical structure capable of performing various roles required in an organic light emitting device depending on substituents have been required.

### Prior Art Documents

### Patent Documents

US Patent No. 4,356,429

### [Disclosure]

### [Technical Problem]

The present application relates to a heterocyclic compound, and an organic light emitting device comprising the same.

### [Technical Solution]

One embodiment of the present application provides a heterocyclic compound represented by the following Chemical Formula 1.

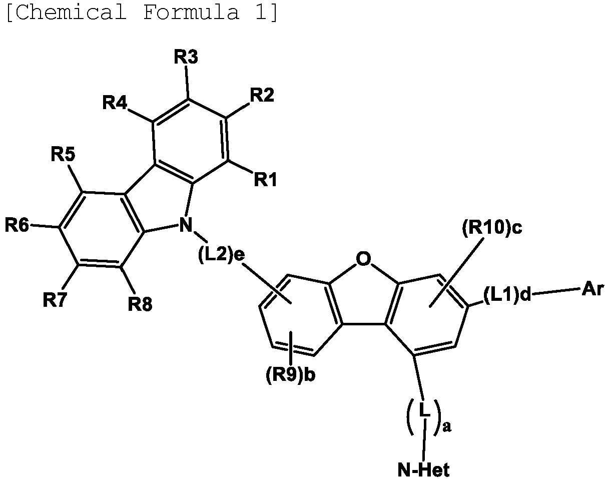

In Chemical Formula 1,
N-Het is a monocyclic or polycyclic heterocyclic group substituted or unsubstituted, and comprising one or more Ns,
R1 to R10 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R" and -NRR', or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring,
L, L1 and L2 are the same as or different from each other, and each independently a direct bond; a substituted or unsubstituted C6 to C60 arylene group; or a substituted or unsubstituted C2 to C60 heteroarylene group,
Ar is a substituted or unsubstituted C1 to C60 alkyl group; a substituted or unsubstituted C6 to C60 aryl group; a substituted or unsubstituted C2 to C60 heteroaryl group; - P(=O)RR'; or -SiRR'R",
R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group,
a, d and e are the same as or different from each other, and each an integer of 0 to 4,
b is an integer of 0 to 3,
c is an integer of 0 to 2, and
when a to e are 2 or greater, substituents in the parentheses are the same as or different from each other.

Another embodiment of the present application provides an organic light emitting device comprising a first electrode; a second electrode provided opposite to the first electrode; and one or more organic material layers provided between the first electrode and the second electrode, wherein one or more layers of the organic material layers comprise the heterocyclic compound represented by Chemical Formula 1.

### [Advantageous Effects]

A compound described in the present specification can be used as a material of an organic material layer of an organic light emitting device. In the organic light emitting device, the compound is capable of performing a role of a hole injection material, a hole transport material, a light emitting material, an electron transport material, an electron injection material or the like. Particularly, the compound can be used as a light emitting material of the organic light emitting device. For example, the compound can be used alone as a light emitting material, or two of the compounds can be used together as a light emitting material, and can be used as a host material of a light emitting layer.

Particularly, by the heterocyclic compound according to the present application having a specific substituent at No. 1 and No. 3 positions of one side benzene ring of the dibenzofuran, and having a carbazole-based substituent on the other side benzene ring, an organic light emitting device comprising the same has properties of excellent lifetime and efficiency.

### [Description of Drawings]

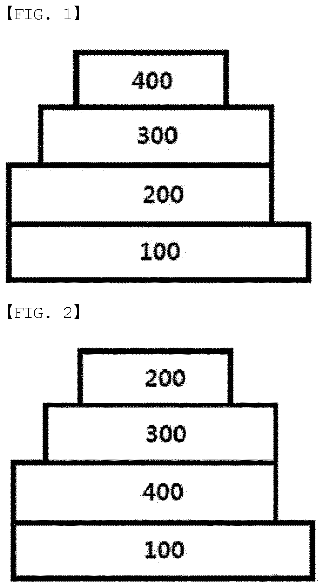

FIG. 1 to FIG. 3 are diagrams each schematically illustrating a lamination structure of an organic light emitting device according to one embodiment of the present application.

### [Reference Numeral]

- 100:: Substrate
- 200:: Anode
- 300:: Organic Material Layer
- 301:: Hole Injection Layer
- 302:: Hole transport Layer
- 303:: Light Emitting Layer
- 304:: Hole Blocking Layer
- 305:: Electron transport Layer
- 306:: Electron Injection Layer
- 400:: Cathode

### [Mode for Disclosure]

Hereinafter, the present application will be described in detail.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0% or a hydrogen content being 100%. In other words, an expression of "substituent X is hydrogen" does not exclude deuterium such as a hydrogen content being 100% or a deuterium content being 0%, and therefore, may mean a state in which hydrogen and deuterium are mixed.

In one embodiment of the present application, deuterium is one of isotopes of hydrogen, is an element having deuteron formed with one proton and one neutron as a nucleus, and may be expressed as hydrogen-2, and the elemental symbol may also be written as D or 2H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 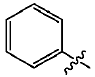 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

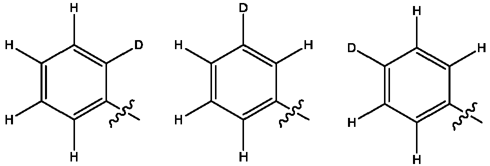

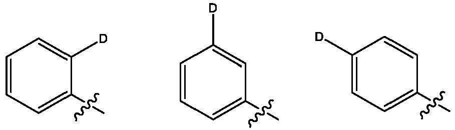

In addition, in one embodiment of the present application, "a phenyl group having a deuterium content of 0%" may mean a phenyl group that does not comprise a deuterium atom, that is, a phenyl group that has 5 hydrogen atoms.

In the present specification, the halogen may be fluorine, chlorine, bromine or iodine.

In the present specification, the alkyl group comprises linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may comprise a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethylbutyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group comprises linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may comprise a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group comprises linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may comprise methoxy, ethoxy, n-propoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group comprises monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may comprise a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group comprises O, S, Se, N or Si as a heteroatom, comprises monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group comprises monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The number of carbon atoms of the aryl group may be from 6 to 60, specifically from 6 to 40 and more specifically from 6 to 25. Specific examples of the aryl group may comprise a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, an indenyl group, an acenaphthylenyl group, a 2,3-dihydro-1H-indenyl group, a fused ring group thereof, and the like, but are not limited thereto.

In the present specification, a fluorenyl group may be substituted, and adjacent substituents may bond to each other to form a ring.

When the fluorenyl group is substituted, the following structural formulae and the like may be included, however, the structure is not limited thereto.

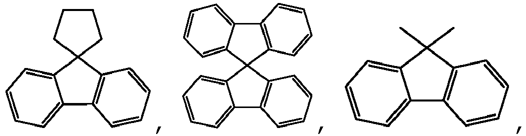

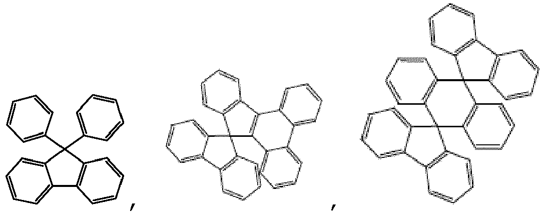

In the present specification, the heteroaryl group comprises S, O, Se, N or Si as a heteroatom, comprises monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may comprise a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, a 5,10-dihydrobenzo[b,e][1,4]azasilinyl group, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may comprise a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except for those that are each a divalent group. In addition, the heteroarylene group means the heteroaryl group having two bonding sites, that is, a divalent group. The descriptions on the heteroaryl group provided above may be applied thereto except for those that are each a divalent group.

In the present specification, the phosphine oxide group is represented by -P(=O)R₁₀₁R₁₀₂, and R₁₀₁ and R₁₀₂ are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the phosphine oxide may comprise a diphenylphosphine oxide group, a dinaphthylphosphine oxide group and the like, but are not limited thereto.

In the present specification, the silyl group is a substituent comprising Si, having the Si atom directly linked as a radical, and is represented by -SiR₁₀₄R₁₀₅R₁₀₆. R₁₀₄ to R₁₀₆ are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may comprise a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

As the aliphatic or aromatic hydrocarbon ring or heteroring that adjacent groups may form, the structures illustrated as the cycloalkyl group, the cycloheteroalkyl group, the aryl group and the heteroaryl group described above may be used except for those that are not a monovalent group.

In the present specification, the term "substitution" means a hydrogen atom bonding to a carbon atom of a compound being changed to another substituent, and the position of substitution is not limited as long as it is a position at which the hydrogen atom is substituted, that is, a position at which a substituent can substitute, and when two or more substituents substitute, the two or more substituents may be the same as or different from each other.

In the present specification, "substituted or unsubstituted" means being substituted with one or more substituents selected from the group consisting of C1 to C60 linear or branched alkyl; C2 to C60 linear or branched alkenyl; C2 to C60 linear or branched alkynyl; C3 to C60 monocyclic or polycyclic cycloalkyl; C2 to C60 monocyclic or polycyclic heterocycloalkyl; C6 to C60 monocyclic or polycyclic aryl; C2 to C60 monocyclic or polycyclic heteroaryl; -SiRR'R";-P(=O)RR'; C1 to C20 alkylamine; C6 to C60 monocyclic or polycyclic arylamine; and C2 to C60 monocyclic or polycyclic heteroarylamine, or being unsubstituted, or being substituted with a substituent linking two or more substituents selected from among the substituents illustrated above, or being unsubstituted, and
R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group.

One embodiment of the present application provides a heterocyclic compound represented by Chemical Formula 1.

Particularly, Chemical Formula 1 is a tri-substituted compound in which a carbazole-based substituent substituting one side benzene ring of the dibenzofuran is a substituent having hole transport characteristics (hole transport character moiety), N-Het substituting a No. 1 position of the other side benzene ring of the dibenzofuran is a substituent having electron transport characteristics (electron transport character moiety), and in addition, an Ar substituent is included at the No. 3 position. In other words, when Ar is not substituted, the No. 3 position of the dibenzofuran becomes relatively electron deficient, and by forming an Ar substituent such as Chemical Formula 1 of the present application at the No. 3 position of the dibenzofuran, electrons become abundant increasing structural stability, and as a result, an organic light emitting device comprising the same has properties of enhancing lifetime and current flow.

In one embodiment of the present application, Chemical Formula 1 may be represented by any one of the following Chemical Formulae 2 to 5.

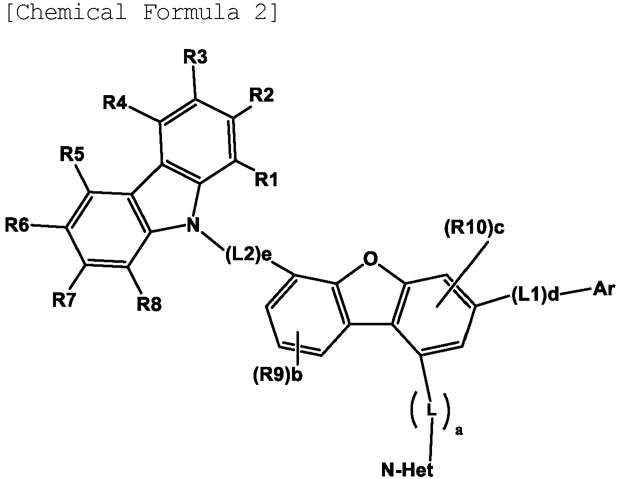

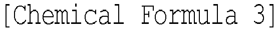

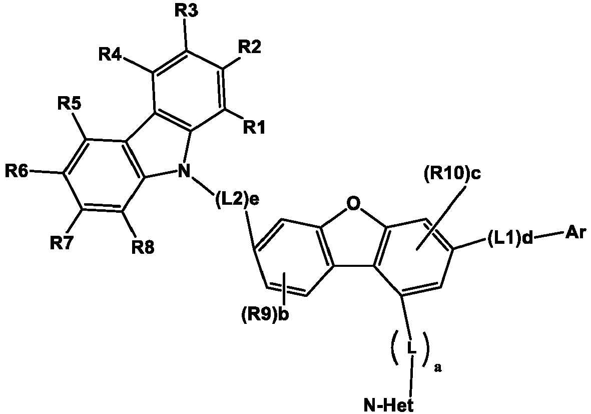

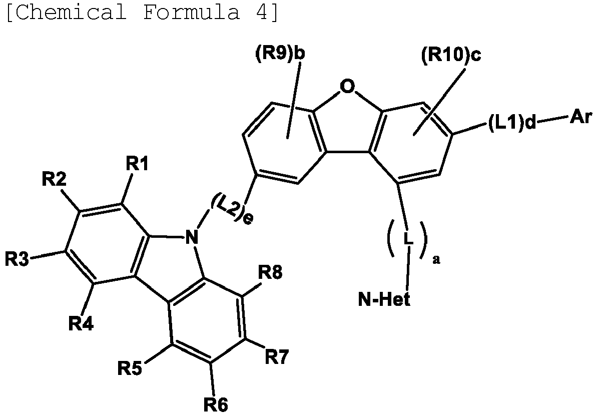

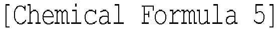

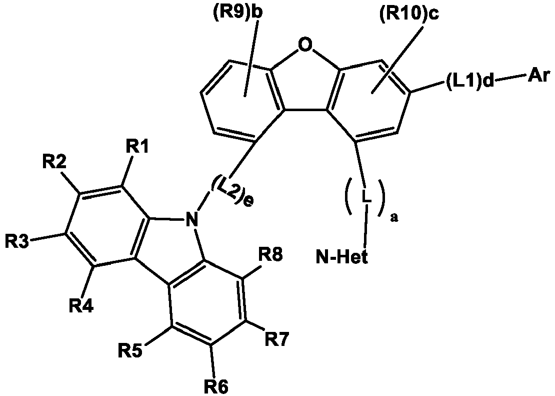

In Chemical Formulae 2 to 5,
R1 to R10, N-Het, Ar, L, L1, L2, a, b, c, d and e have the same definitions as in Chemical Formula 1.

Particularly, in Chemical Formulae 2 and 3, a carbazole-based substituent substituting the dibenzofuran substitutes at the No. 3 or No. 4 position, which tends to have a higher T_{d} (95%) value by approximately 10°C to 40°C compared to cases of substituting other positions, and as a result, particularly superior thermal stability is obtained. In other words, in Chemical Formulae 2 and 3, the No. 3 or No. 4 position is substituted with a carbazole-based substituent, and two substituents are formed at No. 1 and No. 3 positions of the benzene ring on the opposite side, and as a result, the molecular weight increases, and stability of the structure itself increases.

In one embodiment of the present application, Chemical Formula 1 may be represented by the following Chemical Formula 6 or Chemical Formula 7.

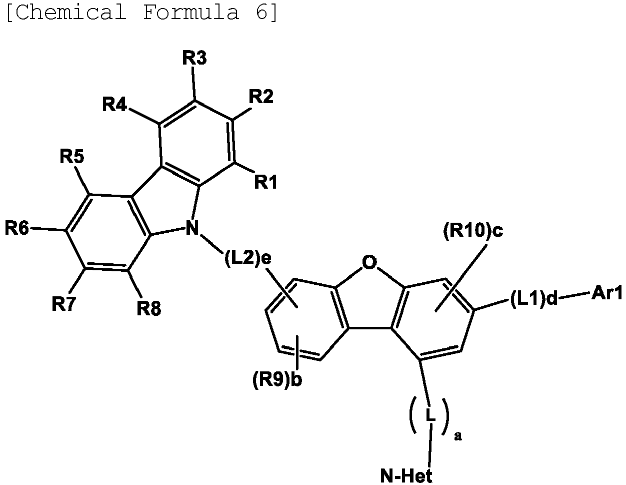

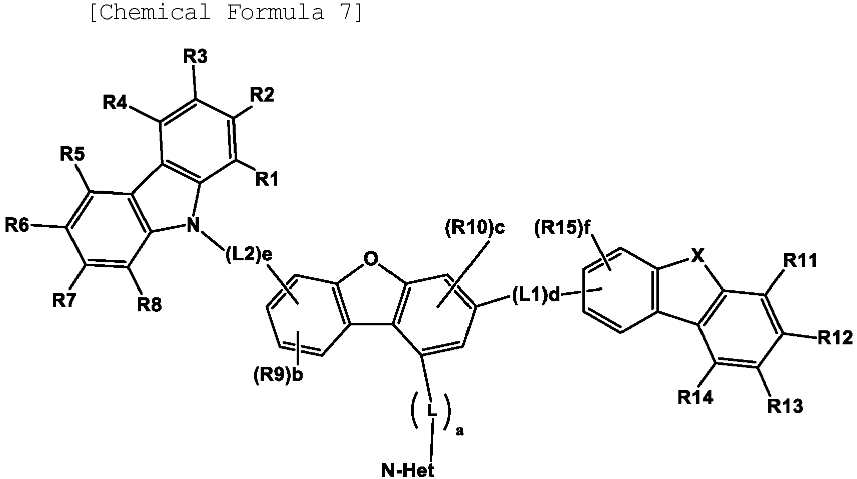

In Chemical Formulae 6 and 7,
R1 to R10, N-Het, L, L1, L2, a, b, c, d and e have the same definitions as in Chemical Formula 1,
Ar1 is a substituted or unsubstituted C6 to C60 aryl group,
X is O or S,
R11 to R15 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R" and -NRR', or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring, and
f is an integer of 0 to 3, and when f is 2 or greater, substituents in the parentheses are the same as or different from each other.

In one embodiment of the present application, L, L1 and L2 are the same as or different from each other, and may be each independently a direct bond; a substituted or unsubstituted C6 to C60 arylene group; or a substituted or unsubstituted C2 to C60 heteroarylene group.

In another embodiment, L, L1 and L2 are the same as or different from each other, and may be each independently a direct bond; a substituted or unsubstituted C6 to C40 arylene group; or a substituted or unsubstituted C2 to C40 heteroarylene group.

In another embodiment, L, L1 and L2 are the same as or different from each other, and may be each independently a direct bond; or a substituted or unsubstituted C6 to C40 monocyclic or polycyclic arylene group.

In another embodiment, L, L1 and L2 are the same as or different from each other, and may be each independently a direct bond; or a substituted or unsubstituted C6 to C20 monocyclic arylene group.

In another embodiment, L, L1 and L2 are the same as or different from each other, and may be each independently a direct bond; or a C6 to C20 monocyclic arylene group.

In another embodiment, L, L1 and L2 are the same as or different from each other, and may be each independently a direct bond; or a phenylene group.

In one embodiment of the present application, R1 to R10 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R" and -NRR', or two or more groups adj acent to each other may bond to each other to form a substituted or unsubstituted C6 to C60 aliphatic or aromatic hydrocarbon ring or a substituted or unsubstituted C2 to C60 aliphatic or aromatic heteroring.

In another embodiment, R1 to R10 are the same as or different from each other, and each independently selected from the group consisting of hydrogen; a substituted or unsubstituted C1 to C60 alkyl group; a substituted or unsubstituted C6 to C60 aryl group; a substituted or unsubstituted C2 to C60 heteroaryl group; -P(=O)RR'; -SiRR'R" and -NRR', or two or more groups adj acent to each other may bond to each other to form a substituted or unsubstituted C6 to C60 aliphatic or aromatic hydrocarbon ring or a substituted or unsubstituted C2 to C60 aliphatic or aromatic heteroring.

In another embodiment, R1 to R10 are the same as or different from each other, and each independently selected from the group consisting of hydrogen; a substituted or unsubstituted C6 to C40 aryl group; and a substituted or unsubstituted C2 to C40 heteroaryl group, or two or more groups adjacent to each other may bond to each other to form a substituted or unsubstituted C6 to C40 aromatic hydrocarbon ring or a substituted or unsubstituted C2 to C40 aromatic heteroring.

In another embodiment, R1 to R10 are the same as or different from each other, and each independently selected from the group consisting of hydrogen; a C6 to C40 aryl group; and a C2 to C40 heteroaryl group unsubstituted or substituted with a C6 to C40 aryl group, or two or more groups adjacent to each other may bond to each other to form a C6 to C40 aromatic hydrocarbon ring unsubstituted or substituted with a C1 to C10 alkyl group or a C6 to C40 aryl group, or a C2 to C40 aromatic heteroring unsubstituted or substituted with a C6 to C40 aryl group.

In another embodiment, R1 to R10 are the same as or different from each other, and each independently selected from the group consisting of hydrogen; a C6 to C20 aryl group; and a C2 to C20 heteroaryl group unsubstituted or substituted with a C6 to C20 aryl group, or two or more groups adjacent to each other may bond to each other to form a C6 to C20 aromatic hydrocarbon ring unsubstituted or substituted with a C1 to C10 alkyl group or a C6 to C20 aryl group, or a C2 to C20 aromatic heteroring unsubstituted or substituted with a C6 to C20 aryl group.

In another embodiment, R1 to R10 are the same as or different from each other, and each independently selected from the group consisting of hydrogen; a phenyl group; a carbazole group unsubstituted or substituted with a phenyl group; a dibenzofuran group; and a dibenzothiophene group, or two or more groups adjacent to each other may bond to each other to form a benzene ring; a benzothiophene ring; a benzofuran ring; an indole ring unsubstituted or substituted with a phenyl group; or an indene ring unsubstituted or substituted with a methyl group or a phenyl group.

In one embodiment of the present application, R9 and R10 may be hydrogen.

In one embodiment of the present application, Ar may be a substituted or unsubstituted C1 to C60 alkyl group; a substituted or unsubstituted C6 to C60 aryl group; a substituted or unsubstituted C2 to C60 heteroaryl group; - P(=O)RR'; or -SiRR'R".

In another embodiment, Ar may be a substituted or unsubstituted C6 to C60 aryl group; or a substituted or unsubstituted C2 to C60 heteroaryl group.

In another embodiment, Ar may be a substituted or unsubstituted C6 to C40 aryl group; or a substituted or unsubstituted C2 to C40 heteroaryl group.

In another embodiment, Ar may be a C6 to C40 monocyclic or polycyclic aryl group; or a C2 to C40 heteroaryl group unsubstituted or substituted with one or more substituents selected from the group consisting of a C6 to C40 aryl group and a C2 to C40 heteroaryl group.

In another embodiment, Ar may be a phenyl group; a biphenyl group; a naphthalenyl group; a dibenzothiophene group unsubstituted or substituted with one or more substituents selected from the group consisting of a phenyl group and a dibenzofuran group; or a dibenzofuran group.

In one embodiment of the present application, N-Het may be a monocyclic or polycyclic heterocyclic group substituted or unsubstituted, and comprising one or more Ns.

In another embodiment, N-Het may be a monocyclic or polycyclic C2 to C60 heterocyclic group substituted or unsubstituted, and comprising one or more and three or less Ns.

In another embodiment, N-Het may be a monocyclic or polycyclic C2 to C40 heterocyclic group substituted or unsubstituted, and comprising one or more and three or less Ns.

In another embodiment, N-Het may be a monocyclic or polycyclic C2 to C40 heterocyclic group unsubstituted or substituted with a C6 to C40 aryl group, and comprising one or more and three or less Ns.

In another embodiment, N-Het may be a triazine group unsubstituted or substituted with a substituent; a substituted or unsubstituted pyrimidine group; a substituted or unsubstituted pyridine group; a substituted or unsubstituted quinoline group; a substituted or unsubstituted 1,10-phenanthroline group; a substituted or unsubstituted 1,7-phenanthroline group; a substituted or unsubstituted quinazoline group; or a substituted or unsubstituted benzimidazole group.

In another embodiment, N-Het may be a triazine group unsubstituted or substituted with one or more substituents selected from the group consisting of a phenyl group, a biphenyl group and a naphthyl group; a pyrimidine group unsubstituted or substituted with one or more substituents selected from the group consisting of a phenyl group, a biphenyl group and a naphthyl group; a pyridine group unsubstituted or substituted with one or more substituents selected from the group consisting of a phenyl group, a biphenyl group and a naphthyl group; a quinoline group unsubstituted or substituted with a phenyl group; a 1,10-phenanthroline group unsubstituted or substituted with a phenyl group; a 1,7-phenanthroline group unsubstituted or substituted with a phenyl group; a quinazoline group unsubstituted or substituted with a phenyl group or a biphenyl group; or a benzimidazole group unsubstituted or substituted with a phenyl group or a biphenyl group.

In one embodiment of the present application, the 1,10-phenanthroline group may be represented by the following Chemical Formula 1-1, and the 1,7-phenanthroline group may be represented by the following Chemical Formula 1-2.

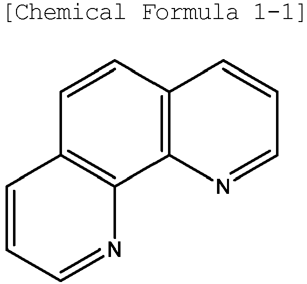

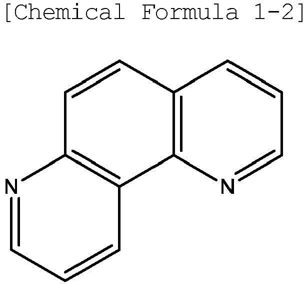

In one embodiment of the present application, R, R' and R" are the same as or different from each other, and may be each independently a substituted or unsubstituted C1 to C60 alkyl group; a substituted or unsubstituted C6 to C60 aryl group; or a substituted or unsubstituted C2 to C60 heteroaryl group.

In another embodiment, R, R' and R" are the same as or different from each other, and may be each independently a substituted or unsubstituted C6 to C60 aryl group.

In another embodiment, R, R' and R" are the same as or different from each other, and may be each independently a substituted or unsubstituted C6 to C60 monocyclic or polycyclic aryl group.

In another embodiment, R, R' and R" are the same as or different from each other, and may be each independently a substituted or unsubstituted C6 to C40 monocyclic aryl group.

In another embodiment, R, R' and R" are the same as or different from each other, and may be each independently a C6 to C20 monocyclic aryl group.

In another embodiment, R, R' and R" may be a phenyl group.

In one embodiment of the present application, Ar1 may be a substituted or unsubstituted C6 to C60 aryl group.

In another embodiment, Ar1 may be a substituted or unsubstituted C6 to C40 monocyclic or polycyclic aryl group.

In another embodiment, Ar1 may be a C6 to C40 monocyclic or polycyclic aryl group.

In another embodiment, Ar1 may be a C6 to C40 monocyclic aryl group.

In another embodiment, Ar1 may be a C6 to C40 polycyclic aryl group.

In another embodiment, Ar1 may be a phenyl group; a biphenyl group; or a naphthyl group.

In one embodiment of the present application, R11 to R15 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R" and -NRR', or two or more groups adj acent to each other may bond to each other to form a substituted or unsubstituted C6 to C60 aliphatic or aromatic hydrocarbon ring or a substituted or unsubstituted C2 to C60 aliphatic or aromatic heteroring.

In another embodiment, R11 to R15 are the same as or different from each other, and may be each independently selected from the group consisting of hydrogen; a substituted or unsubstituted C6 to C60 aryl group; and a substituted or unsubstituted C2 to C60 heteroaryl group.

In another embodiment, R11 to R15 are the same as or different from each other, and may be each independently selected from the group consisting of hydrogen; a substituted or unsubstituted C6 to C40 aryl group; and a substituted or unsubstituted C2 to C40 heteroaryl group.

In another embodiment, R11 to R15 are the same as or different from each other, and may be each independently selected from the group consisting of hydrogen; a C6 to C40 aryl group; and a C2 to C40 heteroaryl group.

In another embodiment, R11 to R15 are the same as or different from each other, and may be each independently selected from the group consisting of hydrogen; a C6 to C20 aryl group; and a C2 to C20 heteroaryl group.

In another embodiment, R11 to R15 are the same as or different from each other, and may be each independently hydrogen; a phenyl group; a dibenzofuran group; or a dibenzothiophene group.

In another embodiment, R15 may be hydrogen.

According to one embodiment of the present application, Chemical Formula 1 may be represented by any one of the following compounds, but is not limited thereto.

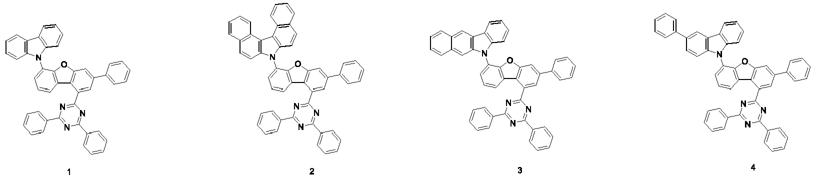

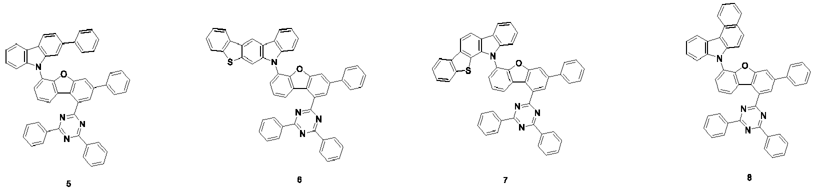

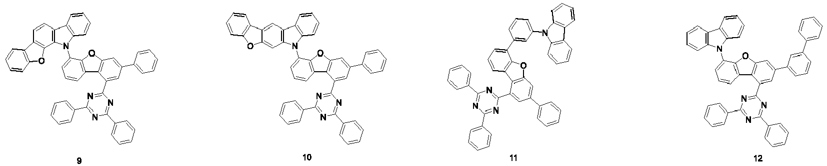

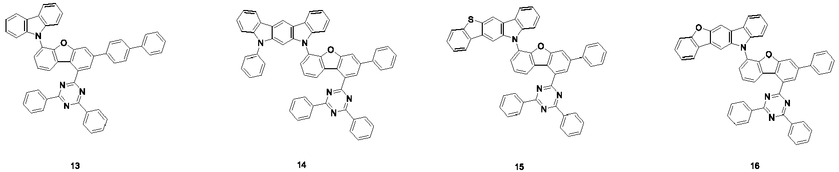

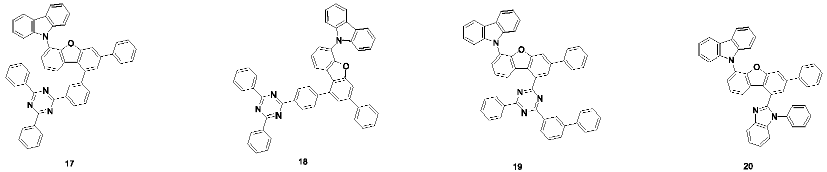

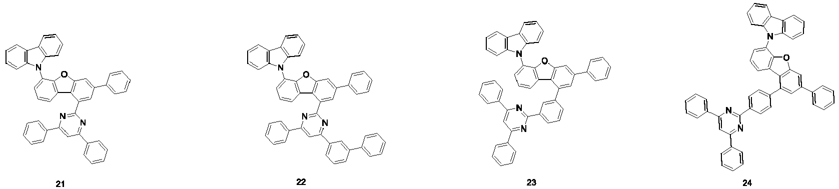

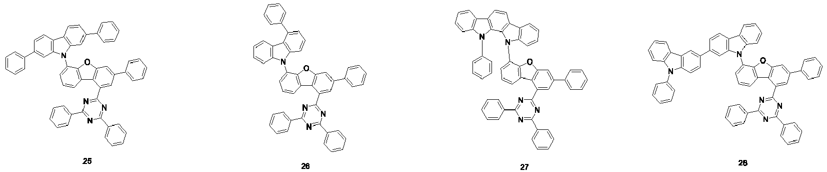

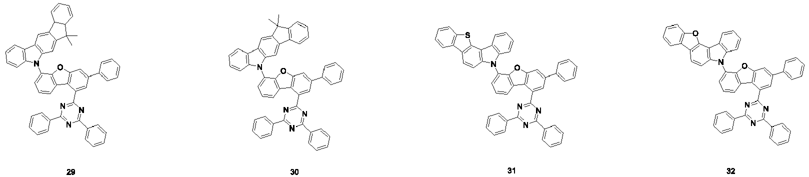

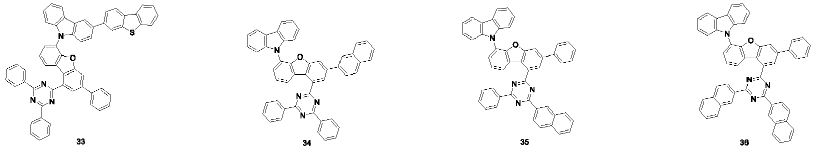

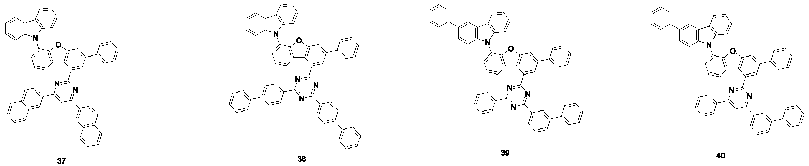

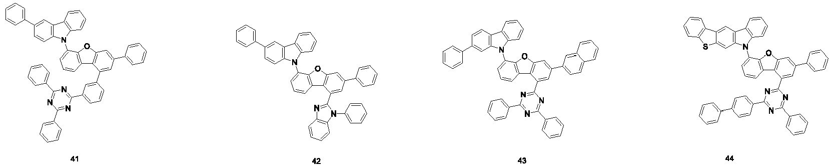

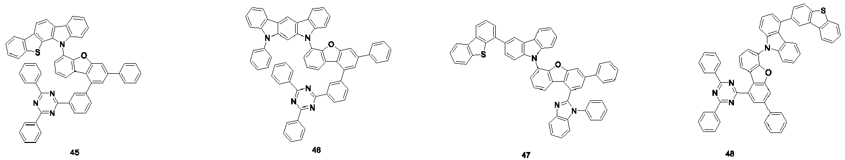

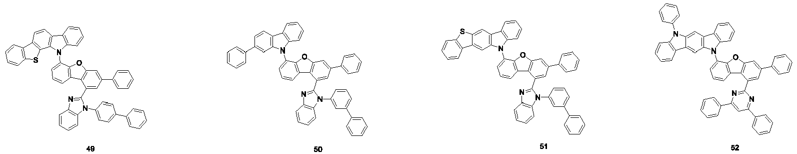

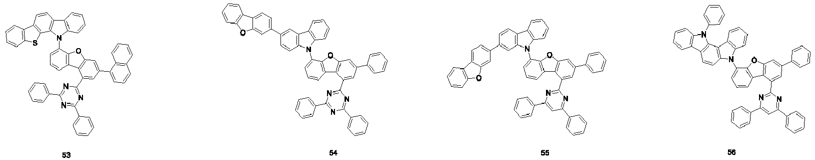

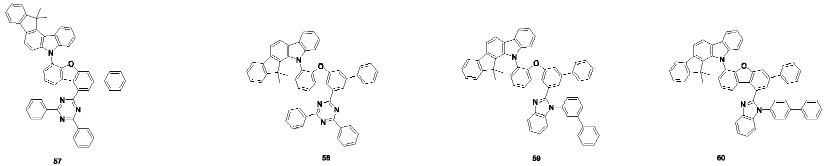

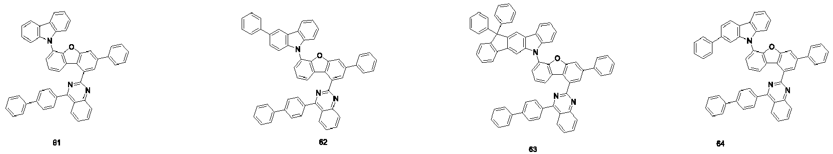

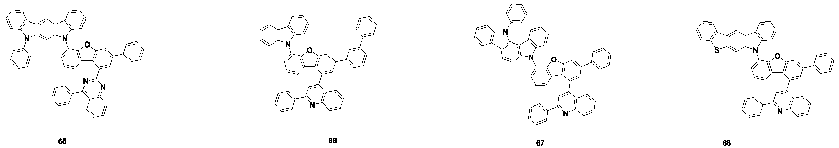

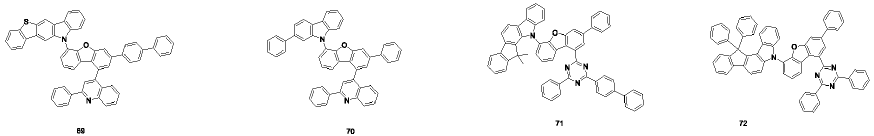

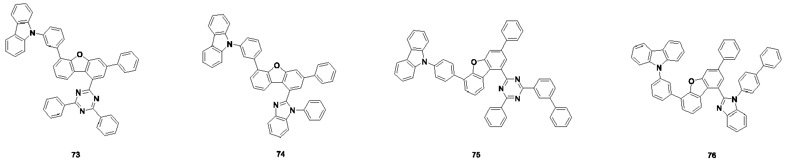

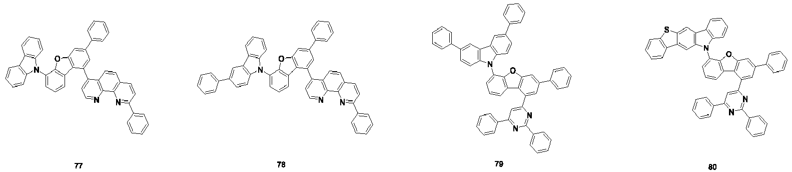

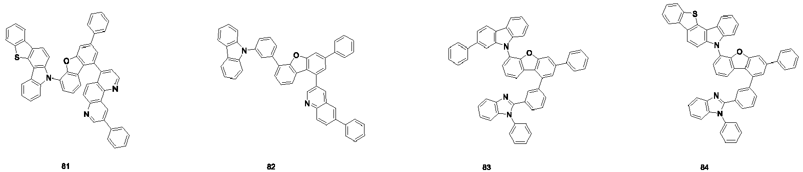

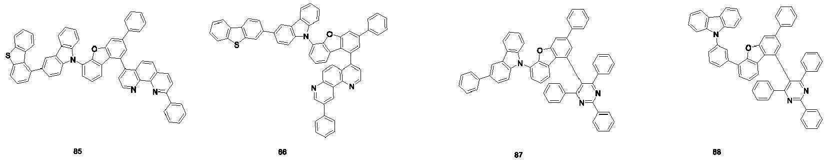

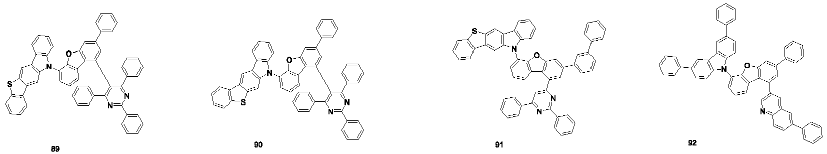

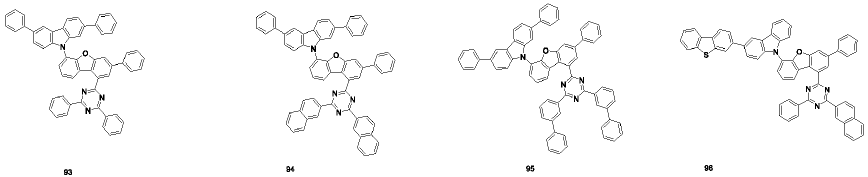

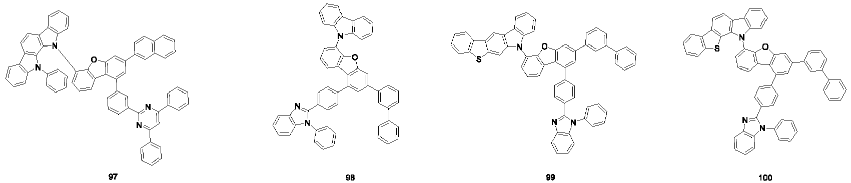

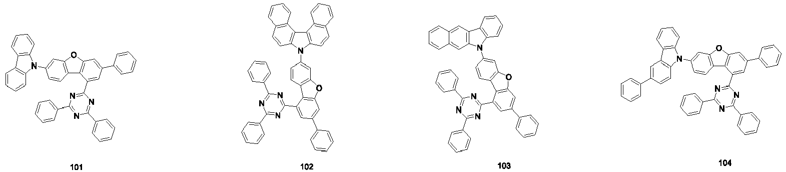

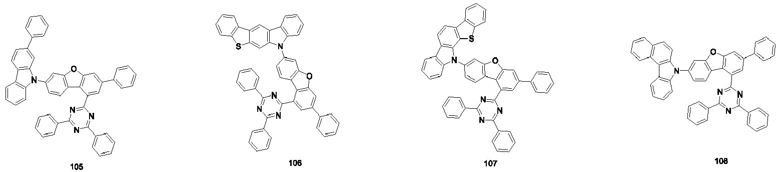

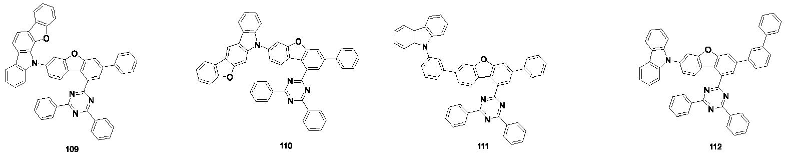

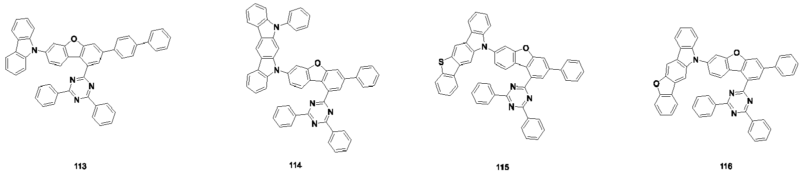

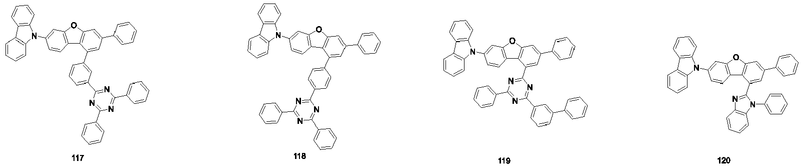

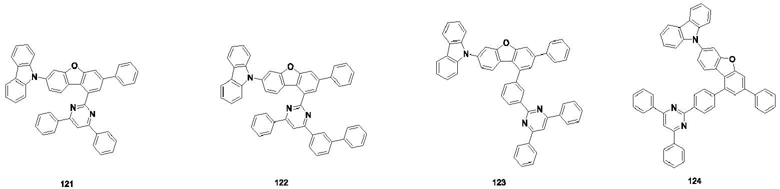

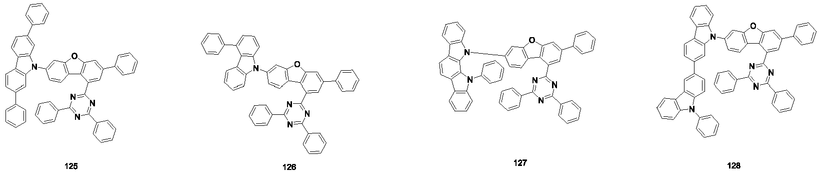

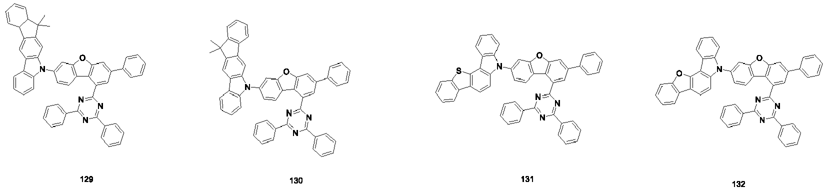

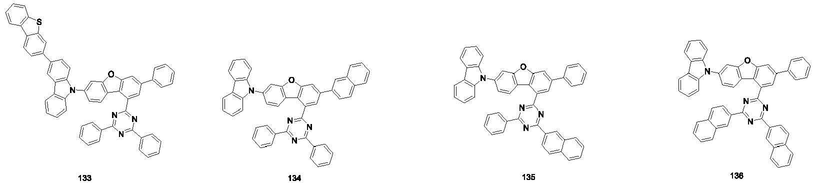

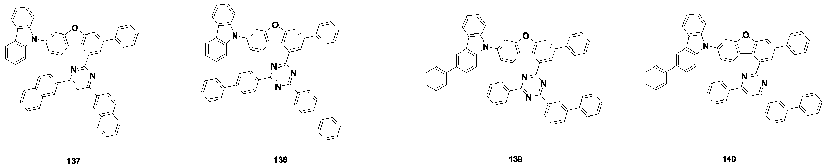

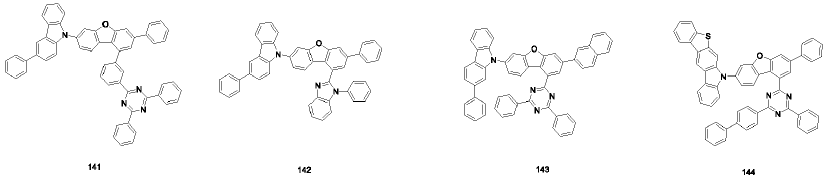

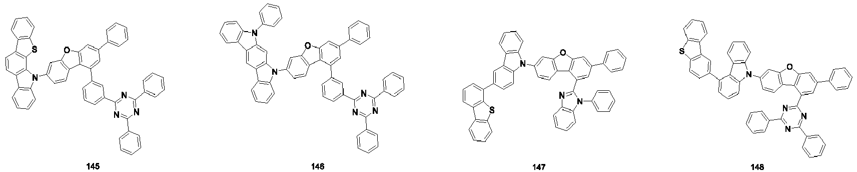

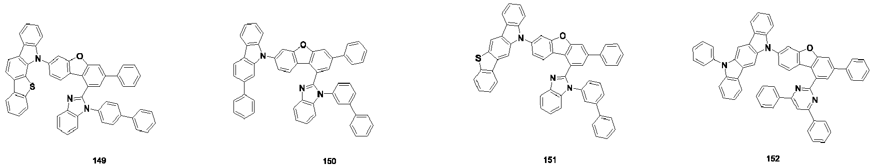

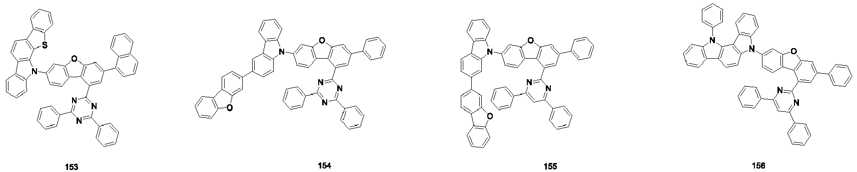

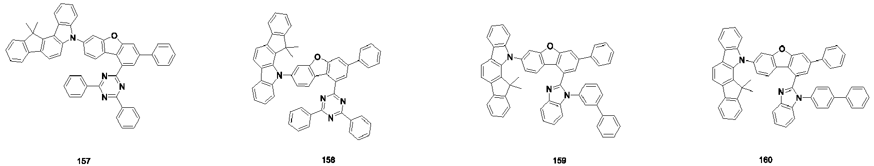

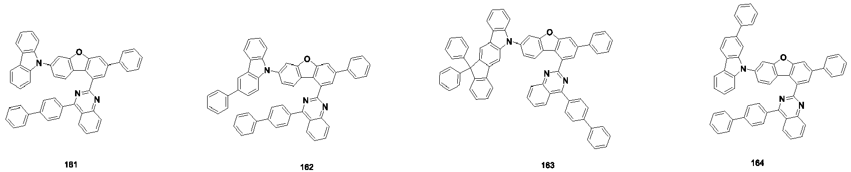

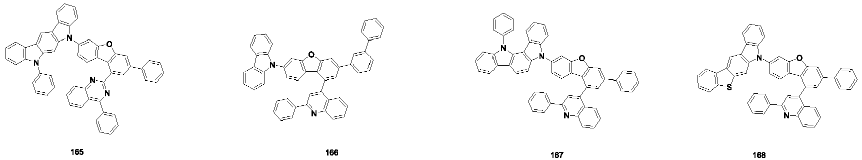

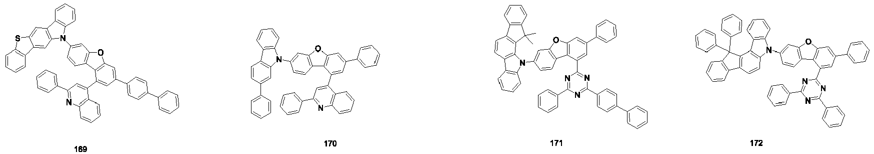

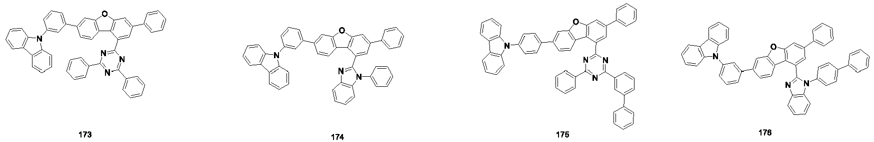

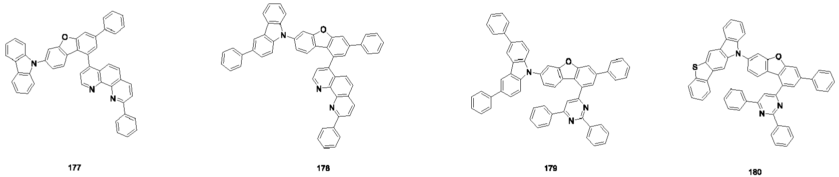

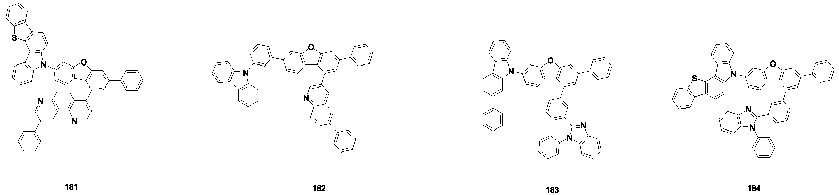

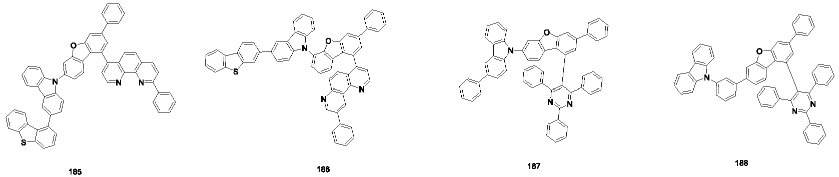

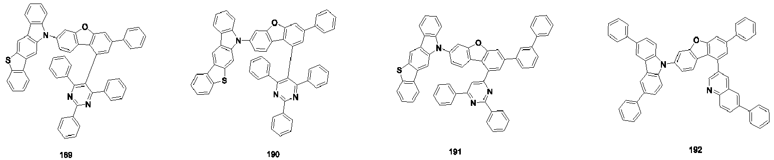

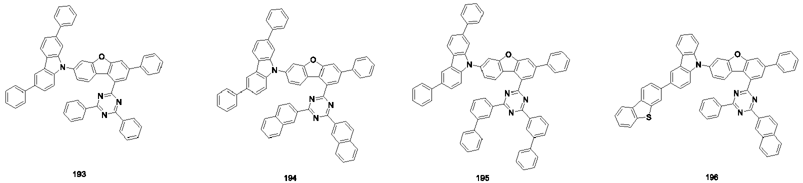

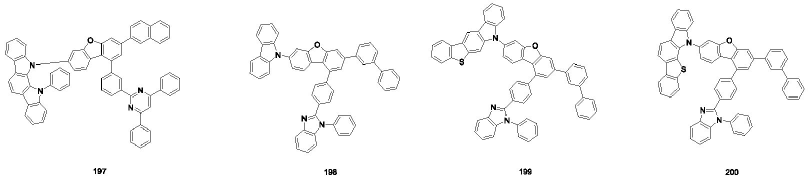

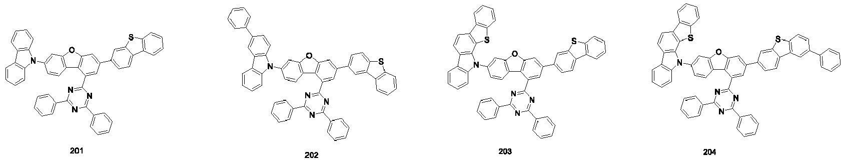

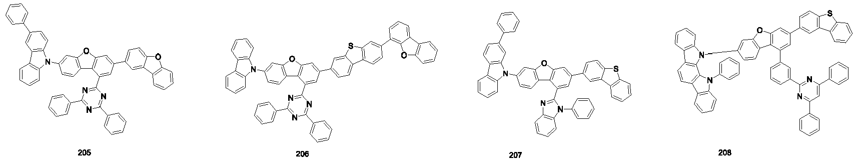

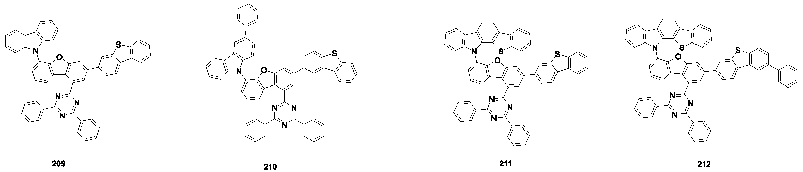

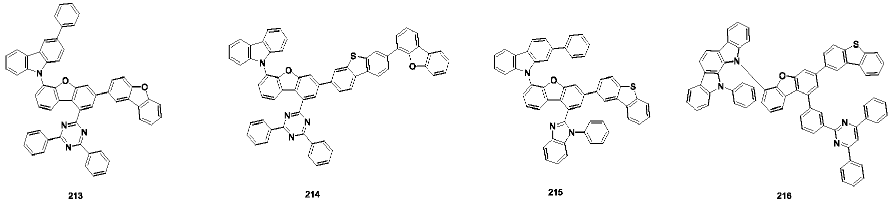

In addition, by introducing various substituents to the structure of Chemical Formula 1, compounds having unique properties of the introduced substituents may be synthesized. For example, by introducing substituents normally used as hole injection layer materials, hole transport layer materials, light emitting layer materials, electron transport layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

In addition, one embodiment of the present application provides an organic light emitting device comprising a first electrode; a second electrode provided opposite to the first electrode; and one or more organic material layers provided between the first electrode and the second electrode, wherein one or more layers of the organic material layers comprise the heterocyclic compound according to Chemical Formula 1.

Another embodiment of the present application provides an organic light emitting device comprising a first electrode; a second electrode provided opposite to the first electrode; and one or more organic material layers provided between the first electrode and the second electrode, wherein one or more layers of the organic material layers comprise one heterocyclic compound according to Chemical Formula 1.

Specific descriptions on the heterocyclic compound represented by Chemical Formula 1 are the same as the descriptions provided above.

In one embodiment of the present application, the first electrode may be an anode, and the second electrode may be a cathode.

In another embodiment, the first electrode may be a cathode, and the second electrode may be an anode.

In one embodiment of the present application, the organic light emitting device may be a blue organic light emitting device, and the heterocyclic compound according to Chemical Formula 1 may be used as a material of the blue organic light emitting device. For example, the heterocyclic compound according to Chemical Formula 1 may be included in a host material of a blue light emitting layer of the blue organic light emitting device.

In one embodiment of the present application, the organic light emitting device may be a green organic light emitting device, and the heterocyclic compound according to Chemical Formula 1 may be used as a material of the green organic light emitting device. For example, the heterocyclic compound according to Chemical Formula 1 may be included in a host material of a green light emitting layer of the green organic light emitting device.

In one embodiment of the present application, the organic light emitting device may be a red organic light emitting device, and the heterocyclic compound according to Chemical Formula 1 may be used as a material of the red organic light emitting device. For example, the heterocyclic compound according to Chemical Formula 1 may be included in a host material of a red light emitting layer of the red organic light emitting device.

The organic light emitting device of the present disclosure may be manufactured using common organic light emitting device manufacturing methods and materials except that one or more of the organic material layers are formed using the heterocyclic compound described above.

The heterocyclic compound may be formed into an organic material layer through a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present disclosure may be formed in a single layer structure, but may be formed in a multilayer structure in which two or more organic material layers are laminated. For example, the organic light emitting device of the present disclosure may have a structure comprising a hole injection layer, a hole transport layer, a light emitting layer, an electron transport layer, an electron injection layer and the like as the organic material layer. However, the structure of the organic light emitting device is not limited thereto, and may comprise a smaller number of organic material layers.

In the organic light emitting device of the present disclosure, the organic material layer may comprise a light emitting layer, and the light emitting layer may comprise the heterocyclic compound.

In another organic light emitting device, the organic material layer comprises a light emitting layer, the light emitting layer comprises a host material, and the host material may comprise the heterocyclic compound.

As another example, the organic material layer comprising the heterocyclic compound comprises the heterocyclic compound represented by Chemical Formula 1 as a host, and an iridiumbased dopant may be used therewith.

In the organic light emitting device of the present disclosure, the organic material layer comprises an electron injection layer or an electron transport layer, and the electron transport layer or the electron injection layer may comprise the heterocyclic compound.

In another organic light emitting device, the organic material layer comprises an electron blocking layer or a hole blocking layer, and the electron blocking layer or the hole blocking layer may comprise the heterocyclic compound.

The organic light emitting device of the present disclosure may further comprise one, two or more layers selected from the group consisting of a light emitting layer, a hole injection layer, a hole transport layer, an electron injection layer, an electron transport layer, an electron blocking layer and a hole blocking layer.

FIG 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

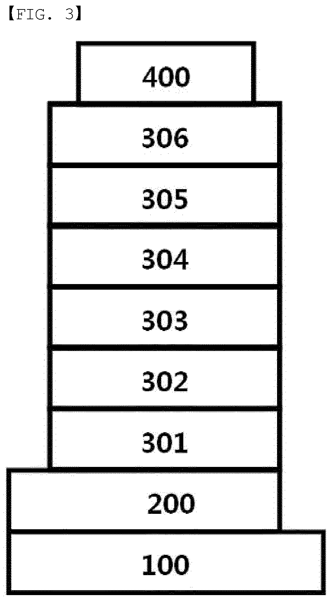

FIG. 3 illustrates a case of the organic material layer being a multilayer. The organic light emitting device according to FIG. 3 comprises a hole injection layer (301), a hole transport layer (302), a light emitting layer (303), a hole blocking layer (304), an electron transport layer (305) and an electron injection layer (306). However, the scope of the present application is not limited to such a lamination structure, and as necessary, layers other than the light emitting layer may not be included, and other necessary functional layers may be further added.

The organic material layer comprising the compound of Chemical Formula 1 may further comprise other materials as necessary.

In the organic light emitting device according to one embodiment of the present application, materials other than the heterocyclic compound of Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material comprise metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material comprise metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino) phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrene-sulfonate) that are conductive polymers having solubility, and the like, may be used.

As the hole transport material, pyrazoline derivatives, arylamine-based derivatives, stilbene derivatives, triphenyldiamine derivatives and the like may be used, and low molecular or high molecular materials may also be used.

As the electron transport material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

When mixing light emitting material hosts, same series hosts may be mixed, or different series hosts may be mixed. For example, any two or more types of materials among n-type host materials or p-type host materials may be selected and used as a host material of a light emitting layer.

The organic light emitting device according to one embodiment of the present application may be a top-emission type, a bottom-emission type or a dual-emission type depending on the materials used.

The heterocyclic compound according to one embodiment of the present application may also be used in an organic electronic device comprising an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example 1> Preparation of Compound 1(D)

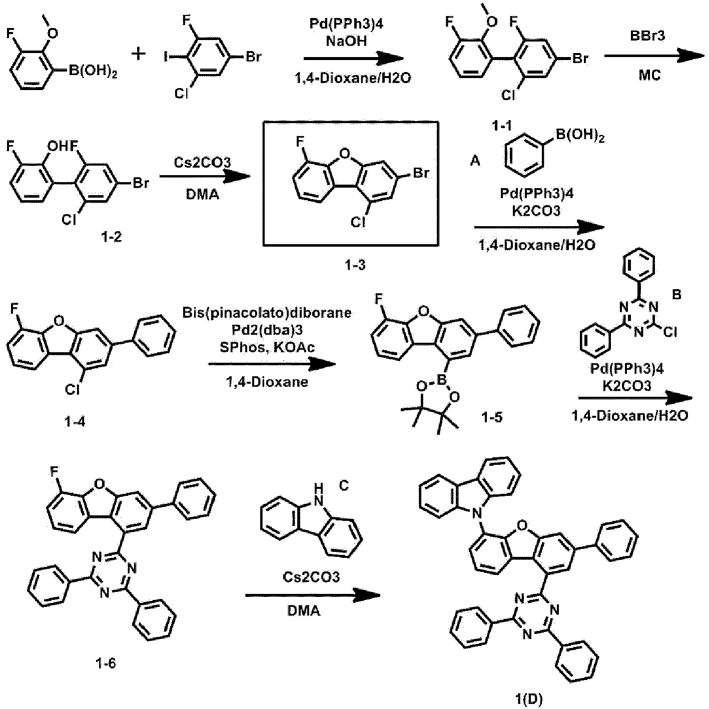

### Preparation of Compound 1-1

In a one-neck round bottom flask (one-neck r.b.f), a mixture of (3-fluoro-2-methoxyphenyl)boronic acid (27.87 g, 164.01 mmol), 5-bromo-1-chloro-3-fluoro-2-iodobenzene (50 g, 149.10 mmol), tetrakis(triphenylphosphine)palladium(0) (8.61 g, 7.46 mmol), sodium hydroxide (11.93 g, 298.20 mmol) and 1,4-dioxane/water (500 mL/150 mL) was refluxed at 120°C.

The result was extracted with dichloromethane, and dried with MgSO₄. The result was silica gel filtered, and then concentrated to obtain Compound 1-1 (49.24 g, 99%).

### Preparation of Compound 1-2

In a one-neck round bottom flask (one-neck r.b.f), a mixture of 4'-bromo-2'-chloro-3,6'-difluoro-2-methoxy-1,1'-biphenyl (49.24 g, 147.62 mmol) and methylene chloride (MC) (500 mL) was cooled to 0°C, BBr₃ (73.96 g, 295.24 mmol) was added dropwise threto, and, after raising the temperature to room temperature (25°C), the result was stirred for 3 hours.

The reaction was terminated with distilled water, and the result was extracted with dichloromethane and dried with MgSO₄. The result was silica gel filtered, and then concentrated to obtain Compound 1-2 (44.81 g, 95%).

### Preparation of Compound 1-3

In a one-neck round bottom flask (one-neck r.b.f), a mixture of 4'-bromo-2'-chloro-3,6'-difluoro-[1,1'-biphenyl]-2-ol (44.81 g, 140.24 mmol), Cs₂CO₃ (91.39 g, 280.48 mmol) and dimethylacetamide (500 mL) was stirred at 180°C. The result was cooled, then filtered, and, after removing the solvent of the filtrate, column purified to obtain Compound 1-3 (36.54 g, 87%).

### Preparation of Compound 1-4

In a one-neck round bottom flask (one-neck r.b.f), a mixture of 3-bromo-1-chloro-6-fluorodibenzo[b,d]furan (36.54 g, 122.00 mmol), phenylboronic acid (16.36 g, 134.20 mmol), tetrakis(triphenylphosphine)palladium(0) (7.05 g, 6.10 mmol), potassium carbonate (33.72 g, 244 mmol) and 1,4-dioxane/water (360 mL/108 mL) was refluxed at 120°C.

The result was extracted with dichloromethane, and dried with MgSO₄. The result was column purified to obtain Compound 1-4 (28.96 g, 80%).

### Preparation of Compound 1-5

In a one-neck round bottom flask (one-neck r.b.f), a mixture of 1-chloro-6-fluoro-3-phenyldibenzo[b,d]furan (28.96 g, 97.60 mmol), bis(pinacolato)diboron (49.57 g, 195.2 mmol), Pd₂(dba)₃ (8.94 g, 9.76 mmol), SPhos (8.01 g, 19.52 mmol), potassium acetate (28.74 g, 29.28 mmol) and 1,4-dioxane (290 mL) was refluxed at 140°C.

The result was extracted with dichloromethane, concentrated, and then silica gel filtered. The result was concentrated, and treated with dichloromethane/methanol to obtain Compound 1-5 (36.38 g, 96%).

### Preparation of Compound 1-6

In a one-neck round bottom flask (one-neck r.b.f), a mixture of 2-(6-fluoro-3-phenyldibenzo[b,d]furan-1-yl)-4,4,5,5-tetramethyl-1,3,2-dioxaborolane (36.38 g, 93.70 mmol), 2-chloro-4,6-diphenyl-1,3,5-triazine (27.59 g, 103.07 mmol), tetrakis(triphenylphosphine)palladium(0) (5.41 g, 4.69 mmol), potassium carbonate (25.73 g, 186.14 mmol) and 1,4-dioxane/water (930 mL/279 mL) was refluxed at 120°C.

Solids obtained by vacuum filtering the result were dissolved in dichlorobenzene, and the result was silica gel filtered and then concentrated to obtain Compound 1-6 (35.15 g, 76%).

### Preparation of Compound 1(D)

In a one-neck round bottom flask (one-neck r.b.f), a mixture of 2-(6-fluoro-3-phenyldibenzo[b,d]furan-1-yl)-4,6-diphenyl-1,3,5-triazine (35.15 g, 71.22 mmol), 9H-carbazole (13.10 g, 78.34 mmol), Cs₂CO₃ (46.41 g, 142.44 mmol) and dimethylacetamide (350 mL) was stirred at 180°C.

Solids obtained by vacuum filtering the result were dissolved in dichlorobenzene, and the result was silica gel filtered and then concentrated to obtain Compound 1(D) (37.42 g, 82%).

Each of target Compound D was synthesized in the same manner as in Preparation Example 1 except that Intermediates A, B and C of the following Table 1 were used.

**[Table 1]**

| Compou nd | A | B | C | D | Yield (1-3 to D) |
|---|---|---|---|---|---|
| 1 | 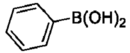 | 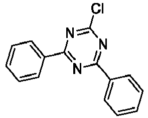 | 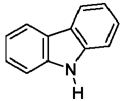 | 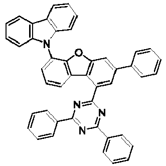 | 48% |
| 4 | 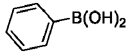 | 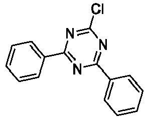 | 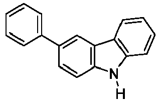 | 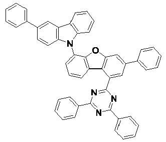 | 78% |
| 5 | 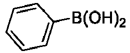 | 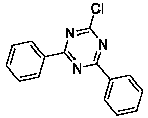 | 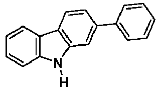 | 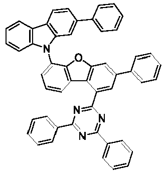 | 68% |
| 6 | 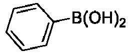 | 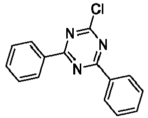 | 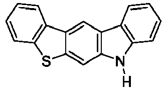 | 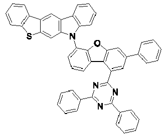 | 59% |
| 7 | 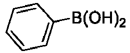 | 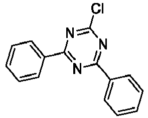 | 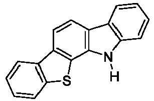 | 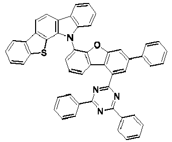 | 48% |
| 9 | 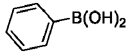 | 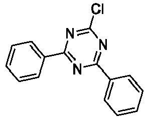 | 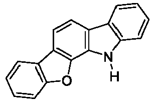 | 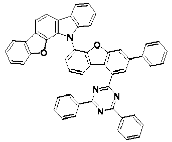 | 46% |
| 10 | 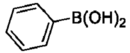 | 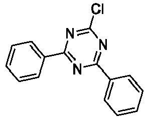 | 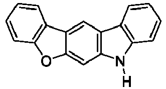 | 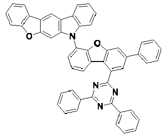 | 87% |
| 14 | 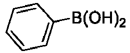 | 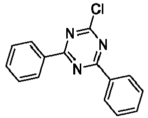 | 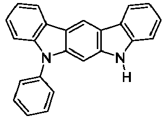 | 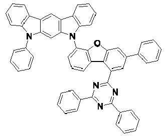 | 90% |
| 16 | 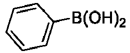 | 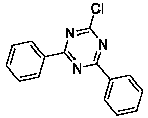 | 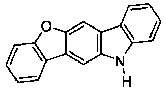 | 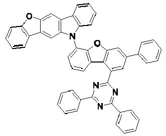 | 96% |
| 22 | 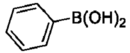 | 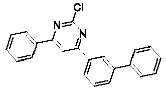 | 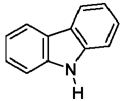 | 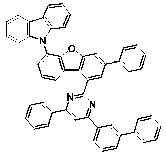 | 86% |
| 25 | 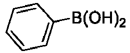 | 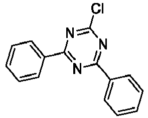 | 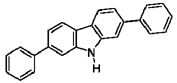 | 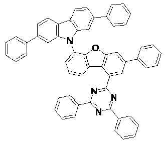 | 72% |
| 27 | 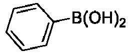 | 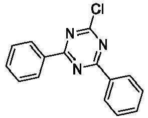 | 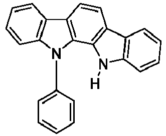 | 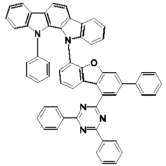 | 74% |
| 29 | 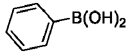 | 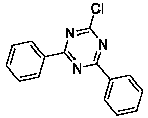 | 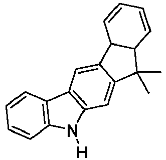 | 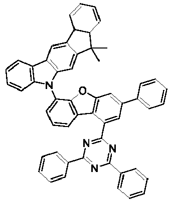 | 89% |
| 31 | 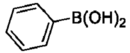 | 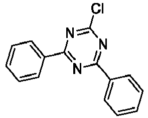 | 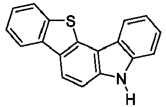 | 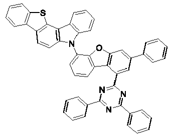 | 67% |
| 33 | 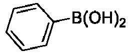 | 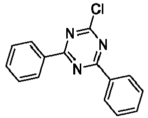 | 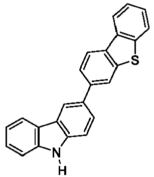 | 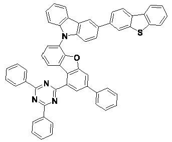 | 88% |
| 38 | 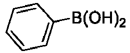 | 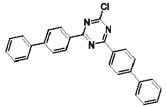 | 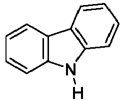 | 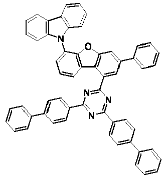 | 90% |
| 44 | 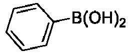 | 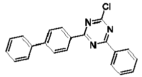 | 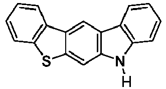 | 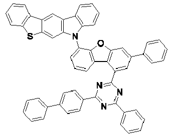 | 83% |
| 46 | 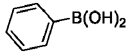 | 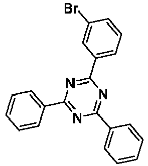 | 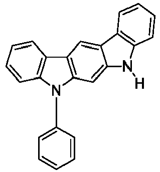 | 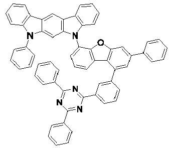 | 39% |
| 48 | 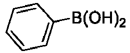 | 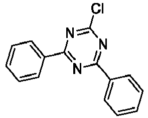 | 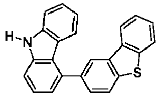 | 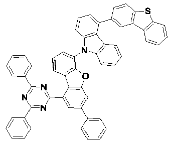 | 72% |
| 54 | 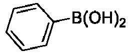 | 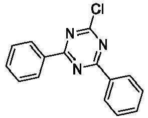 | 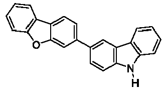 | 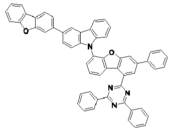 | 56% |
| 56 | 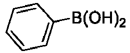 | 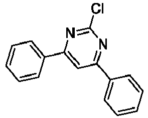 | 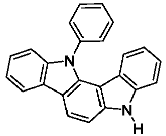 | 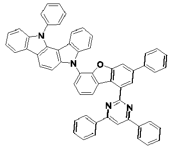 | 86% |
| 58 | 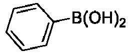 | 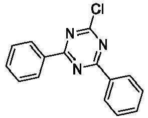 | 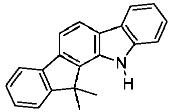 | 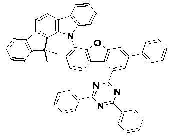 | 74% |
| 64 | 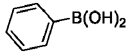 | 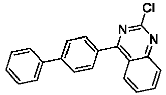 | 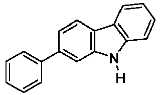 | 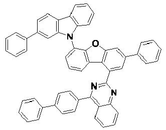 | 83% |
| 66 | 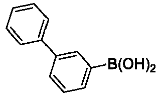 | 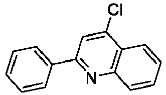 | 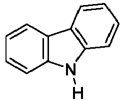 | 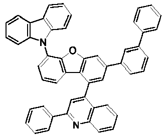 | 86% |
| 68 | 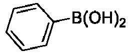 | 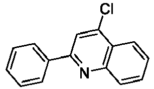 | 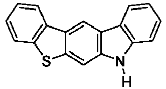 | 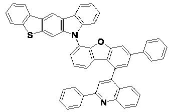 | 77% |
| 71 | 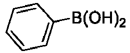 | 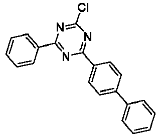 | 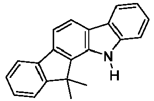 | 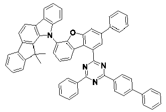 | 53% |
| 73 | 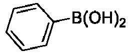 | 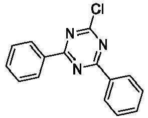 | 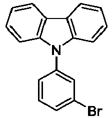 | 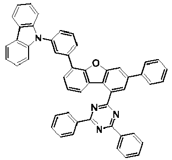 | 86% |
| 83 | 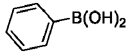 | 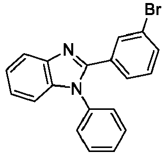 | 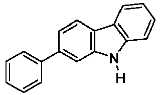 | 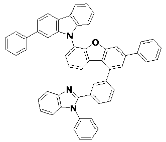 | 71% |
| 86 | 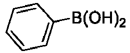 | 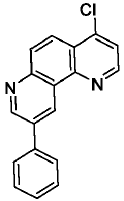 | 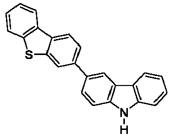 | 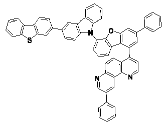 | 48% |
| 90 | 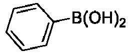 | 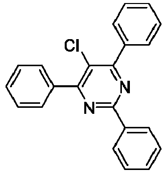 | 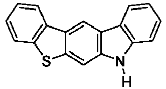 | 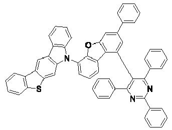 | 73% |
| 92 | 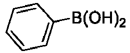 | 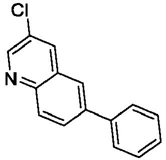 | 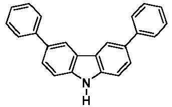 | 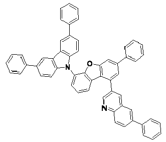 | 46% |
| 96 | 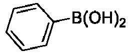 | 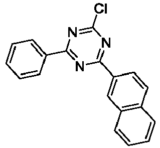 | 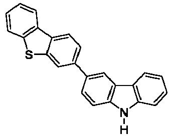 | 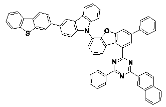 | 59% |
| 100 | 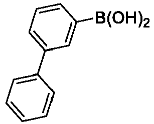 | 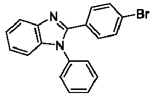 | 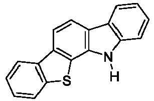 | 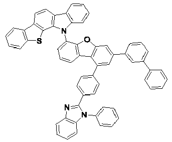 | 39% |
| 210 | 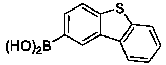 | 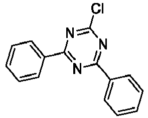 | 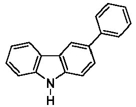 | 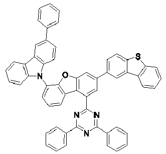 | 78% |
| 211 | 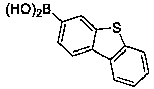 | 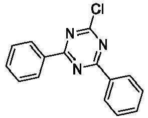 | 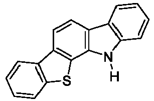 | 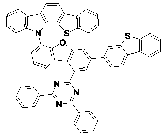 | 83% |
| 213 | 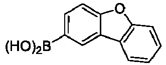 | 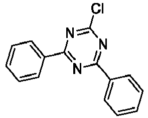 | 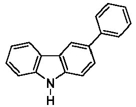 | 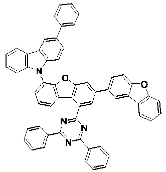 | 79% |
| 215 | 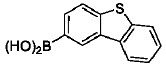 | 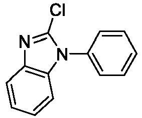 | 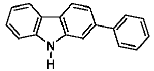 | 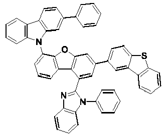 | 65% |

### [Preparation Example 2] Preparation of Compound 101 (E)

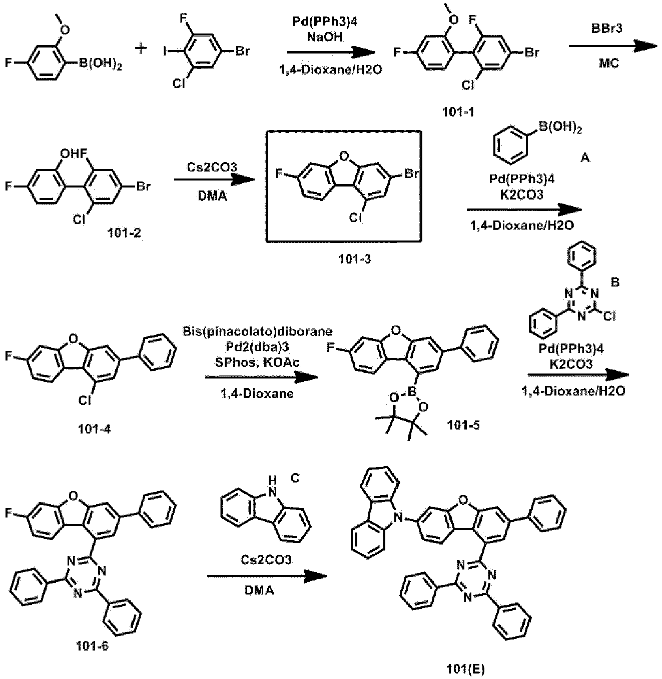

### Preparation of Compound 101-1

In a one-neck round bottom flask (one-neck r.b.f), a mixture of (4-fluoro-2-methoxyphenyl)boronic acid (27.87 g, 164.01 mmol), 5-bromo-1-chloro-3-fluoro-2-iodobenzene (50 g, 149.10 mmol), tetrakis(triphenylphosphine)palladium(0) (8.61 g, 7.46 mmol), sodium hydroxide (11.93 g, 298.20 mmol) and 1,4-dioxane/water (500 mL/150 mL) was refluxed at 120°C.

The result was extracted with dichloromethane, and dried with MgSO₄. The result was silica gel filtered, and then concentrated to obtain Compound 101-1 (43.27 g, 87%).

### Preparation of Compound 101-2

In a one-neck round bottom flask (one-neck r.b.f), a mixture of 4-bromo-2-chloro-4',6-difluoro-2'-methoxy-1,1'-biphenyl (43.27 g, 129.72 mmol) and methylene chloride (MC) (430 mL) was cooled to 0°C, BBr₃ (64.99 g, 259.44 mmol) was added dropwise thereto, and, after raising the temperature to room temperature, the result was stirred for 3 hours.

The reaction was terminated with distilled water, and the result was extracted with dichloromethane and dried with MgSO₄. The result was silica gel filtered, and then concentrated to obtain Compound 101-2 (40.20 g, 97%).

### Preparation of Compound 101-3

In a one-neck round bottom flask (one-neck r.b.f), a mixture of 4'-bromo-2'-chloro-4,6'-difluoro-[1,1'-biphenyl]-2-ol (40.20 g, 134.21 mmol), Cs₂CO₃ (87.46 g, 268.42 mmol) and dimethylacetamide (400 mL) was stirred at 180°C. The result was cooled, then filtered, and, after removing the solvent of the filtrate, column purified to obtain Compound 101-3 (36.18 g, 90%) .

### Preparation of Compound 101-4

In a one-neck round bottom flask (one-neck r.b.f), a mixture of 3-bromo-1-chloro-7-fluorodibenzo[b,d]furan (36.18 g, 120.79 mmol), phenylboronic acid (16.20 g, 132.87 mmol), tetrakis(triphenylphosphine)palladium(0) (6.98 g, 6.04 mmol), potassium carbonate (33.39 g, 241.58 mmol) and 1,4-dioxane/water (360 mL/108 mL) was refluxed at 120°C.

The result was extracted with dichloromethane, and dried with MgSO₄. The result was column purified to obtain Compound 101-4 (33.69 g, 94%).

### Preparation of Compound 101-5

In a one-neck round bottom flask (one-neck r.b.f), a mixture of 1-chloro-7-fluoro-3-phenyldibenzo[b,d]furan (33.69 g, 113.54 mmol), bis(pinacolato)diboron (88.16 g, 227.08 mmol), Pd₂(dba)₃ (10.40 g, 11.35 mmol), SPhos (9.32 g, 22.71 mmol), potassium acetate (33.43 g, 340.62 mmol) and 1,4-dioxane (340 mL) was refluxed at 140°C.

The result was extracted with dichloromethane, concentrated, and then silica gel filtered. The result was concentrated, and treated with dichloromethane/methanol to obtain Compound 101-5 (38.79 g, 88%).

### Preparation of Compound 101-6

In a one-neck round bottom flask (one-neck r.b.f), a mixture of 2-(6-fluoro-3-phenyldibenzo[b,d]furan-1-yl)-4,4,5,5-tetramethyl-1,3,2-dioxaborolane (38.79 g, 99.91 mmol), 2-chloro-4,6-diphenyl-1,3,5-triazine (29.42 g, 109.90 mmol), tetrakis(triphenylphosphine)palladium(0) (5.77 g, 5.00 mmol), potassium carbonate (27.62 g, 199.82 mmol) and 1,4-dioxane/water (380 mL/114 mL) was refluxed at 120°C.

Solids obtained by vacuum filtering the result were dissolved in dichlorobenzene, and the result was silica gel filtered and then concentrated to obtain Compound 101-6 (45.36 g, 92%).

### Preparation of Compound 101 (E)

In a one-neck round bottom flask (one-neck r.b.f), a mixture of 2-(6-fluoro-3-phenyldibenzo[b,d]furan-1-yl)-4,6-diphenyl-1,3,5-triazine (45.36 g, 91.91 mmol), 9H-carbazole (16.91 g, 101.10 mmol), Cs₂CO₃ (59.89 g, 183.82 mmol) and dimethylacetamide (450 mL) was stirred at 180°C.

Solids obtained by vacuum filtering the result were dissolved in dichlorobenzene, and the result was silica gel filtered and then concentrated to obtain Compound 101(E) (46.52 g, 79%).

Each of target Compound E was synthesized in the same manner as in Preparation Example 2 except that Intermediates A, B and C of the following Table 2 were used.

**[Table 2]**

| Compou nd | A | B | C | E | Yield (101-3 to E) |
|---|---|---|---|---|---|
| 101 | 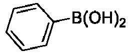 | 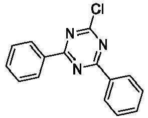 | 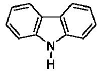 | 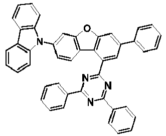 | 60% |
| 103 | 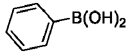 | 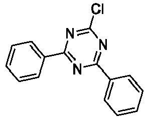 | 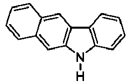 | 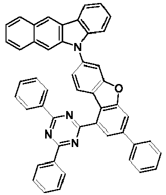 | 87% |
| 104 | 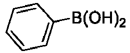 | 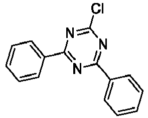 | 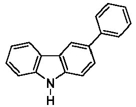 | 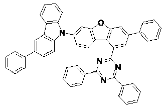 | 79% |
| 106 | 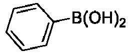 | 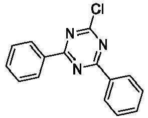 | 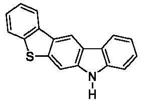 | 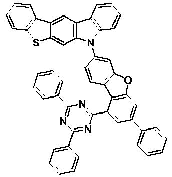 | 68% |
| 109 | 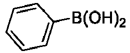 | 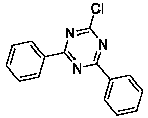 | 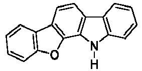 | 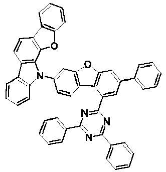 | 66% |
| 110 | 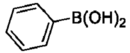 | 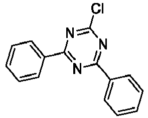 | 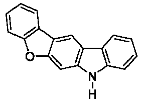 | 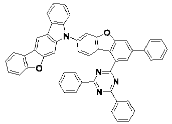 | 83% |
| 112 | 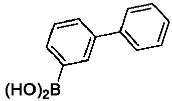 | 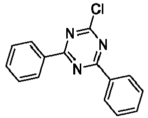 | 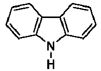 | 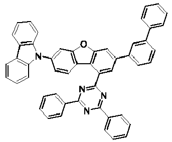 | 76% |
| 114 | 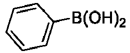 | 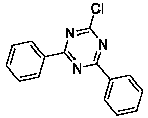 | 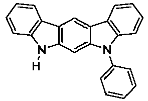 | 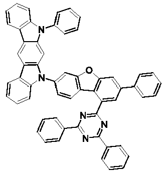 | 70% |
| 116 | 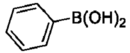 | 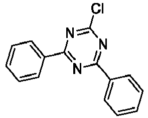 | 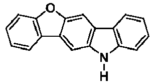 | 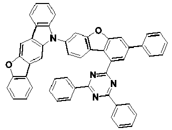 | 83% |
| 117 | 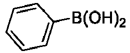 | 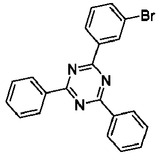 | 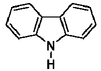 | 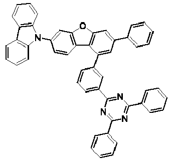 | 91% |
| 123 | 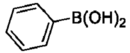 | 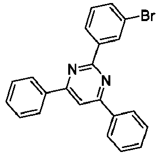 | 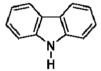 | 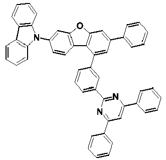 | 88% |
| 127 | 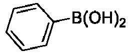 | 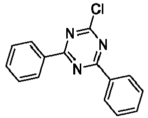 | 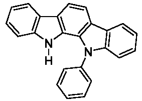 | 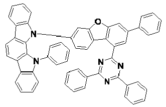 | 49% |
| 129 | 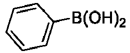 | 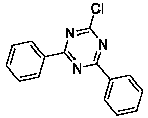 | 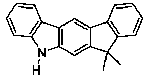 | 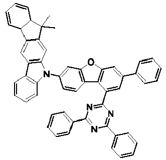 | 77% |
| 131 | 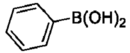 | 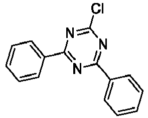 | 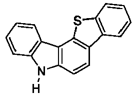 | 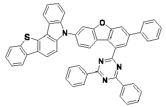 | 61% |
| 133 | 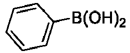 | 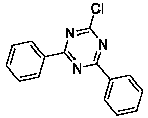 | 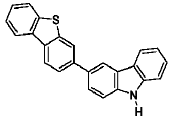 | 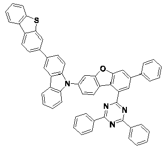 | 69% |
| 135 | 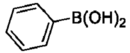 | 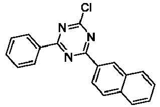 | 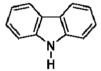 | 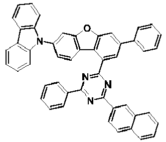 | 84% |
| 139 | 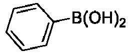 | 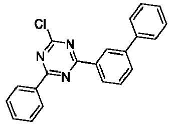 | 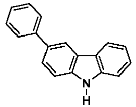 | 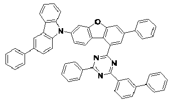 | 59% |
| 142 | 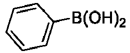 | 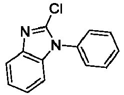 | 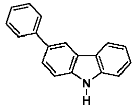 | 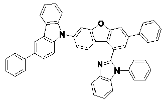 | 81% |
| 144 | 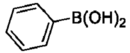 | 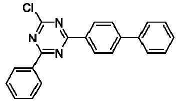 | 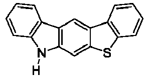 | 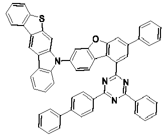 | 70% |
| 145 | 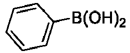 | 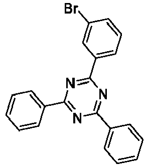 | 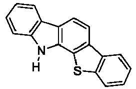 | 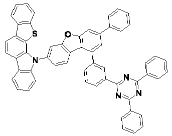 | 76% |
| 146 | 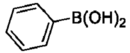 | 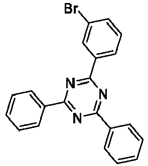 | 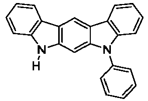 | 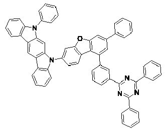 | 89% |
| 151 | 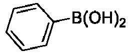 | 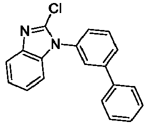 | 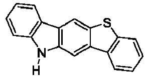 | 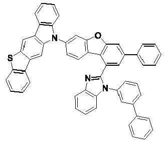 | 93% |
| 156 | 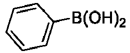 | 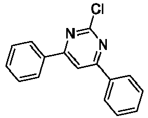 | 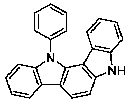 | 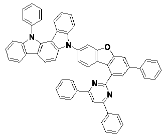 | 76% |
| 157 | 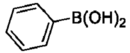 | 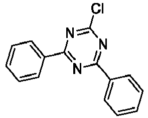 | 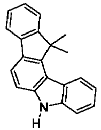 | 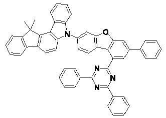 | 85% |
| 160 | 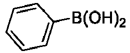 | 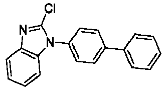 | 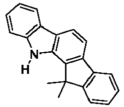 | 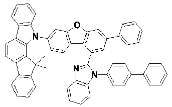 | 74% |
| 163 | 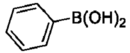 | 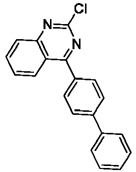 | 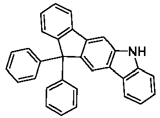 | 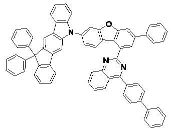 | 48% |
| 165 | 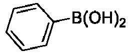 | 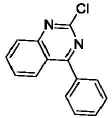 | 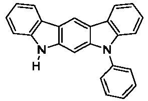 | 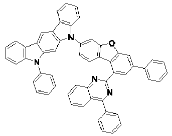 | 68% |
| 168 | 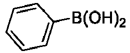 | 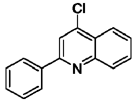 | 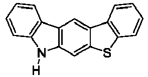 | 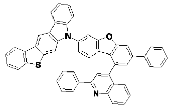 | 76% |
| 171 | 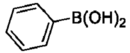 | 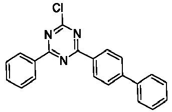 | 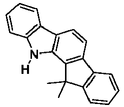 | 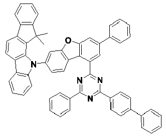 | 79% |
| 176 | 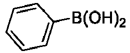 | 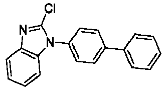 | 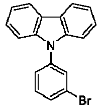 | 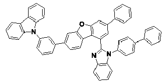 | 81% |
| 177 | 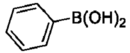 | 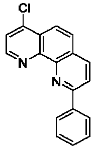 | 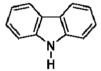 | 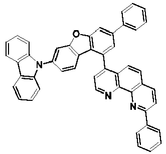 | 76% |
| 184 | 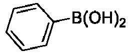 | 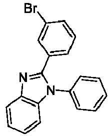 | 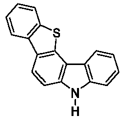 | 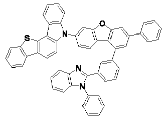 | 83% |
| 188 | 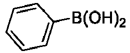 | 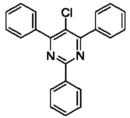 | 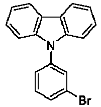 | 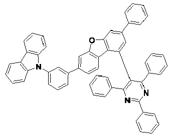 | 72% |
| 190 | 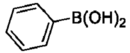 | 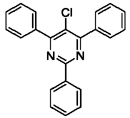 | 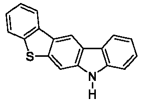 | 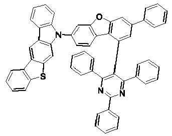 | 74% |
| 195 | 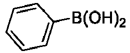 | 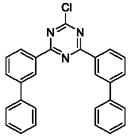 | 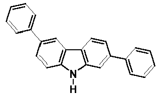 | 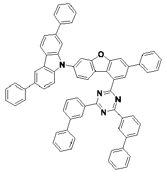 | 46% |
| 200 | 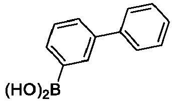 | 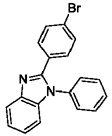 | 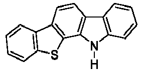 | 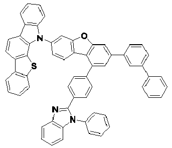 | 53% |
| 201 | 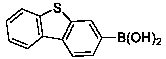 | 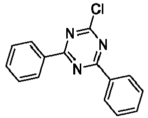 | 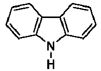 | 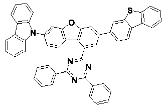 | 88% |
| 204 | 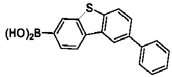 | 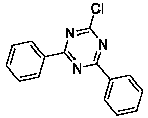 | 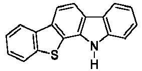 | 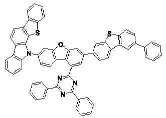 | 84% |
| 205 | 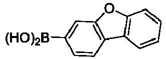 | 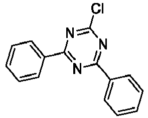 | 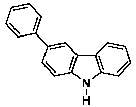 | 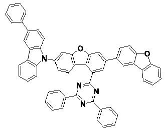 | 73% |

Heterocyclic compounds corresponding to Chemical Formula 1 other than the compounds described in Preparation Examples 1 and 2, and Table 1 and Table 2 were also prepared in the same manner as in the preparation examples described above.

Synthesis identification data of the compounds prepared above are as described in the following [Table 3] and [Table 4] .

**[Table 3]**

| Comp ound | FD-Mass | Comp ound | FD-Mass |
|---|---|---|---|
| 1 | m/z=640.75 (C45H28N4O=640.23) | 2 | m/z=740.87 (C53H32N4O=740.26) |
| 3 | m/z=690.81(C49H30N40=690.24) | 4 | m/z=716.84 (C51H32N40=716.26) |
| 5 | m/z=716.84 (C51H32N40=716.26) | 6 | m/z=746.89 (C51H30N4OS=746.21) |
| 7 | m/z=746.89 (C51H30N4OS=746.21) | 8 | m/z=690.81(C49H30N40=690.24) |
| 9 | m/z=730.83 (C51H30N4O2=730.24) | 10 | m/z=730.83 (C51H30N4O2=730.24) |
| 11 | m/z=716.84 (C51H32N40=716.26) | 12 | m/z=716.84 (C51H32N40=716.26) |
| 13 | m/z=716.84 (C51H32N40=716.26) | 14 | m/z=805.94 (C57H35N5O=805.28) |
| 15 | m/z=746.89 (C51H30N4OS=746.21) | 16 | m/z=730.83 (C51H30N4O2=730.24) |
| 17 | m/z=716.84 (C51H32N40=716.26) | 18 | m/z=716.84 (C51H32N40=716.26) |
| 19 | m/z=716.84 (C51H32N40=716.26) | 20 | m/z=601.71(C43H27N30=601.22) |
| 21 | m/z=639.76(C46H29N3O=639.23) | 22 | m/z=715.86(C52H33N30=715.26) |
| 23 | m/z=715.86(C52H33N30=715.26) | 24 | m/z=715.86(C52H33N30=715.26 |
| 25 | m/z=792.94 (C57H36N40=792.29) | 26 | m/z=716.84 (C51H32N40=716.26) |
| 27 | m/z=805.94 (C57H35N5O=805.28) | 28 | m/z=882.04(C63H39N50=881.32) |
| 29 | m/z=758.93 (C54H38N4O=758.30) | 30 | m/z=756.91(C54H36N40=756.29) |
| 31 | m/z=746.89 (C51H30N4OS=746.21) | 32 | m/z=730.83 (C51H30N4O2=730.24) |
| 33 | m/z=822.99 (C57H34N4O=822.25) | 34 | m/z=690.81(C49H30N40=690.24) |
| 35 | m/z=690.81(C49H30N40=690.24) | 36 | m/z=740.87 (C53H32N4O=740.26) |
| 37 | m/z=739.88 (C54H33N3O=739.26) | 38 | m/z=792.94 (C57H36N40=792.29) |
| 39 | m/z=792.94 (C57H36N40=792.29) | 40 | m/z=791.95 (C58H37N30=791.29) |
| 41 | m/z=792.94 (C57H36N40=792.29) | 42 | m/z=677.81(C49H31N30=677.25) |
| 43 | m/z=766.90(C55H34N40=766.27) | 44 | m/z=822.99 (C57H34N4OS=822.25) |
| 45 | m/z=822.99 (C57H34N4OS=822.25) | 46 | m/z=882.04(C63H39N50=881.32) |
| 47 | m/z=783.95 (C55H33N3OS=783.23) | 48 | m/z=822.99 (C57H34N4OS=822.25 |
| 49 | m/z=783.95 (C55H33N3OS=783.23) | 50 | m/z=753.91(C55H35N30=753.28) |
| 51 | m/z=783.95 (C55H33N3OS=783.23) | 52 | m/z=804.95 (C58H36N4O=804.29) |
| 53 | m/z=796.95 (C55H32N4OS=796.23) | 54 | m/z=806.93 (C57H34N4O2=806.27) |
| 55 | m/z=805.94 (C58H35N3O2=805.27) | 56 | m/z=804.95 (C58H36N4O=804.29) |
| 57 | m/z=756.91(C54H36N40=756.29) | 58 | m/z=756.91(C54H36N40=756.29) |
| 59 | m/z=793.97 (C58H39N30=793.31) | 60 | m/z=793.97 (C58H39N30=793.31) |
| 61 | m/z=689.82 (C50H31N30=689.25) | 62 | m/z=765.92 (C56H35N30=765.28) |
| 63 | m/z=930.12(C69H43N30=929.34) | 64 | m/z=765.92 (C56H35N30=765.28) |
| 65 | m/z=778.92 (C56H34N4O=778.27) | 66 | m/z=688.83 (C51H32N20=688.25) |
| 67 | m/z=777.93 (C57H35N3O=777.28) | 68 | m/z=765.92 (C51H30N2OS=718.21) |
| 69 | m/z=794.97 (C57H34N2OS=794.24) | 70 | m/z=688.83 (C51H32N20=688.25) |
| 71 | m/z=833.01(C60H40N40=832.32) | 72 | m/z=881.05(C64H40N40=880.32) |
| 73 | m/z=716.84 (C51H32N40=716.26) | 74 | m/z=677.81(C49H31N30=677.25) |
| 75 | m/z=792.94 (C57H36N40=792.29) | 76 | m/z=753.91(C55H35N30=753.28) |
| 77 | m/z=663.78 (C48H29N30=663.23) | 78 | m/z=739.88 (C54H33N3O=739.26) |
| 79 | m/z=791.95 (C58H37N30=791.29) | 80 | m/z=753.91(C52H31N3OS=745.22) |
| 81 | m/z=769.92 (C54H31N3OS=969.22) | 82 | m/z=688.83 (C51H32N20=688.25) |
| 83 | m/z=753.91(C55H35N30=753.28) | 84 | m/z=783.95 (C55H33N3OS=783.23) |
| 85 | m/z=846.02(C60H35N3OS=845.25) | 86 | m/z=846.02(C60H35N3OS=845.25) |
| 87 | m/z=791.95 (C58H37N30=791.29) | 88 | m/z=791.95 (C58H37N30=791.29) |
| 89 | m/z=822.00(C58H35N3OS=821.25) | 90 | m/z=822.00(C58H35N3OS=821.25) |
| 91 | m/z=822.00(C58H35N3OS=821.25) | 92 | m/z=764.93 (C57H36N20=764.28) |
| 93 | m/z=792.94 (C57H36N40=792.29) | 94 | m/z=893.06(C65H40N4O=892.32) |
| 95 | m/z=945.14(C69H44N40=944.35) | 96 | m/z=873.05(C61H36N4OS=872.26) |
| 97 | m/z=931.11(C68H42N40=930.34) | 98 | m/z=753.91(C55H35N30=753.28) |
| 99 | m/z=860.05(C61H37N3OS=859.27) | 100 | m/z=860.05(C61H37N3OS=859.27) |
| 101 | m/z=640.75 (C45H26N4O=640.23) | 102 | m/z=740.87 (C53H32N4O=740.26) |
| 103 | m/z=690.81(C51H30N4OS=690.24) | 104 | m/z=716.84 (C51H32N40=716.26) |
| 105 | m/z=730.83 (C51H32N40=716.26) | 106 | m/z=746.89 (C51H30N4OS=746.21) |
| 107 | m/z=746.89 (C51H30N4OS=746.21) | 108 | m/z=690.81(C51H32N40=690.24) |
| 109 | m/z=730.83 (C51H30N4O2=730.24) | 110 | m/z=730.83 (C51H30N4O2=730.24) |
| 111 | m/z=716.84 (C51H32N40=716.26) | 112 | m/z=716.84 (C51HH32N40=716.26) |
| 113 | m/z=716.84 (C51H32N40=716.26) | 114 | m/z=805.94 (C57H35N5O=805.28) |
| 115 | m/z=746.89 (C51H30N4OS=746.21) | 116 | m/z=730.83 (C51H30N4O2=730.24) |
| 117 | m/z=716.84 (C51H32N40=716.26) | 118 | m/z=716.84 (C51H32N40=716.26) |
| 119 | m/z=716.84 (C51H32N40=716.26) | 120 | m/z=601.71(C43H27N30=601.22) |
| 121 | m/z=639.76(C46H29N3O=639.23) | 122 | m/z=715.86(C52H33N30=715.26) |
| 123 | m/z=715.86(C52H33N30=715.26) | 124 | m/z=715.86(C52H33N30=715.26 |
| 125 | m/z=792.94 (C57H36N40=792.29) | 126 | m/z=716.84 (C51H32N40=716.26) |
| 127 | m/z=805.94 (C57H35N5O=805.28) | 128 | m/z=882.04(C63H39N50=881.32) |
| 129 | m/z=758.93 (C54H38N4O=758.30) | 130 | m/z=756.91(C54H36N40=756.29) |
| 131 | m/z=746.89 (C51H30N4OS=746.21) | 132 | m/z=730.83 (C51H30N4O2=730.24) |
| 133 | m/z=822.99 (C57H34N4O=822.25) | 134 | m/z=690.81(C49H30N40=690.24) |
| 135 | m/z=690.81(C49H30N40=690.24) | 136 | m/z=740.87 (C53H32N4O=740.26) |
| 137 | m/z=739.88 (C54H33N3O=739.26) | 138 | m/z=792.94 (C57H36N40=792.29) |
| 139 | m/z=792.94 (C57H36N40=792.29) | 140 | m/z=791.95 (C58H37N30=791.29) |
| 141 | m/z=792.94 (C57H36N40=792.29) | 142 | m/z=677.81(C49H31N30=677.25) |
| 143 | m/z=766.90(C55H34N40=766.27) | 144 | m/z=822.99 (C57H34N4OS=822.25) |
| 145 | m/z=822.99 (C57H34N4OS=822.25) | 146 | m/z=882.04(C63H39N50=881.32) |
| 147 | m/z=783.95 (C55H33N3OS=783.23) | 148 | m/z=822.99 (C57H34N4OS=822.25 |
| 149 | m/z=783.95 (C55H33N3OS=783.23) | 150 | m/z=753.91(C55H35N30=753.28) |
| 151 | m/z=783.95 (C55H33N3OS=783.23) | 152 | m/z=804.95 (C58H36N4O=804.29) |
| 153 | m/z=796.95 (C55H32N4OS=796.23) | 154 | m/z=806.93 (C57H34N4O2=806.27) |
| 155 | m/z=805.94 (C58H35N3O2=805.27) | 156 | m/z=804.95 (C58H36N4O=804.29) |
| 157 | m/z=756.91(C54H36N40=756.29) | 158 | m/z=756.91(C54H36N40=756.29) |
| 159 | m/z=793.97 (C58H39N30=793.31) | 160 | m/z=793.97 (C58H39N30=793.31) |
| 161 | m/z=689.82 (C50H31N30=689.25) | 162 | m/z=765.92 (C56H35N30=765.28) |
| 163 | m/z=930.12(C69H43N30=929.34) | 164 | m/z=765.92 (C56H35N30=765.28) |
| 165 | m/z=778.92 (C56H34N4O=778.27) | 166 | m/z=688.83 (C51H32N20=688.25) |
| 167 | m/z=777.93 (C57H35N3O=777.28) | 168 | m/z=765.92 (C51H30N2OS=718.21) |
| 169 | m/z=794.97 (C57H34N2OS=794.24) | 170 | m/z=688.83 (C51H32N20=688.25) |
| 171 | m/z=833.01(C60H40N40=832.32) | 172 | m/z=881.05(C64H40N40=880.32) |
| 173 | m/z=716.84 (C51H32N40=716.26) | 174 | m/z=677.81(C49H31N30=677.25) |
| 175 | m/z=792.94 (C57H36N40=792.29) | 176 | m/z=753.91(C55H35N30=753.28) |
| 177 | m/z=663.78 (C48H29N30=663.23) | 178 | m/z=739.88 (C54H33N3O=739.26) |
| 179 | m/z=791.95 (C58H37N30=791.29) | 180 | m/z=753.91(C52H31N3OS=745.22) |
| 181 | m/z=769.92 (C54H31N3OS=969.22) | 182 | m/z=688.83 (C51H32N20=688.25) |
| 183 | m/z=753.91(C55H35N30=753.28) | 184 | m/z=783.95 (C55H33N3OS=783.23) |
| 185 | m/z=846.02(C60H35N3OS=845.25) | 186 | m/z=846.02(C60H35N3OS=845.25) |
| 187 | m/z=791.95 (C58H37N30=791.29) | 188 | m/z=791.95 (C58H37N30=791.29) |
| 189 | m/z=822.00(C58H35N3OS=821.25) | 190 | m/z=822.00(C58H35N3OS=821.25) |
| 191 | m/z=822.00(C58H35N3OS=821.25) | 192 | m/z=764.93 (C57H36N20=764.28) |
| 193 | m/z=792.94 (C57H36N40=792.29) | 194 | m/z=893.06(C65H40N4O=892.32) |
| 195 | m/z=945.14(C69H44N40=944.35) | 196 | m/z=873.05(C61H36N4OS=872.26) |
| 197 | m/z=931.11(C68H42N40=930.34) | 198 | m/z=753.91(C55H35N30=753.28) |
| 199 | m/z=860.05(C61H37N3OS=859.27) | 200 | m/z=860.05(C61H37N3OS=859.27) |
| 201 | m/z=746.89 (C51H30N4OS=746.21) | 202 | m/z=822.99 (C57H34N4OS=822.25) |
| 203 | m/z=853.03(C57H32N4OS2=852.20) | 204 | m/z=929.13(C63H36N4OS2=928.23) |
| 205 | m/z=806.93 (C57H34N4O2=806.27) | 206 | m/z=913.07(C63H36N4O2S=912.26) |
| 207 | m/z=783.95 (C55H33N3OS=783.23) | 208 | m/z=987.19(C70H42N4OS=986.31) |
| 209 | m/z=746.89 (C51H30N4OS=746.21) | 210 | m/z=822.99 (C57H34N4OS=822.25) |
| 211 | m/z=853.03(C57H32N4OS2=852.20) | 212 | m/z=929.13(C63H36N4OS2=928.23) |
| 213 | m/z=806.93 (C57H34N4O2=806.27) | 214 | m/z=913.07(C63H36N4O2S=912.26) |
| 215 | m/z=783.95 (C55H33N3OS=783.23) | 216 | m/z=987.19(C70H42N4OS=986.31) |

**[Table 4]**

| Compound | ¹H NMR (CDCl₃, 200 **Mz)** |
|---|---|
| 1 | δ=8.55 (1H, d), 8.36 (4H, m), 8.19 (1H, d), 7.94 (2H, d), 7.75~7.86 (4H, m), 7.31~7.58 (14H, m), 7.16~7.20 (2H, m) |
| 4 | δ=8.55 (1H, d), 8.36 (4H, m), 7.75~7.99 (11H, m), 7.31~7.50 (15H, m), 7.16 (1H, t) |
| 5 | δ=8.55 (1H, d), 8.31~8.36 (5H, m), 7.86~7.94 (4H, m), 7.74~7.81 (6H, m), 7.31~7.50 (15H, m), 7.16 (1H, t) |
| 6 | δ=8.55 (1H, d), 8.45 (1H, d), 8.36 (4H, m), 7.93~7.94 (3H, d), 7.75~7.86 (6H, m), 7.31~7.56 (14H, m), 7.16 (1H, t) |
| 7 | δ=8.55 (1H, d), 8.45 (1H, d), 8.36 (4H, m), 8.05 (1H, d), 7.93~7.94 (3H, d), 7.86 (1H, s), 7.75~7.81 (3H, m), 7.31~7.60 (15H, m), 7.16 (1H, t) |
| 9 | δ=8.55 (1H, d), 8.36 (4H, m), 7.94~7.98 (3H, m), 7.75~7.86 (5H, m), 7.31~7.54 (16H, m), 7.16 (1H, t) |
| 10 | δ=8.55 (1H, d), 8.36 (4H, m), 7.94~7.98 (3H, m), 7.86 (1H, d), 7.75~7.81 (3H, m), 7.31~7.54 (17H, m), 7.16 (1H, t) |
| 14 | δ=8.55 (1H, d), 8.36 (4H, m), 8.19 (1H, d), 7.94 (2H, d), 7.86 (1H, s), 7.75~7.81 (3H, m), 7.31~7.62 (20H, m), 7.16~7.20 (2H, m) |
| 16 | δ=8.55 (1H, d), 8.36 (4H, m), 7.94~7.98 (3H, m), 7.86 (1H, s), 7.75~7.81 (3H, m), 7.31~7.54 (17H, m), 7.16 (1H, t) |
| 22 | δ=8.55 (1H, d), 8.19~8.23 (2H, m), 7.94 (6H, m), 7.73~7.86 (7H, m), 7.31~7.61 (15H, m), 7.16~7.20 (2H, m) |
| 25 | δ=8.62 (1H, d), 8.31~8.36 (4H, m), 8.22 (1H, d), 7.86~7.94 (3H, m), 7.74~7.81 (9H, m), 7.41~7.50 (16H, m), 7.31 (1H, d) |
| 27 | δ=8.55 (2H, d), 8.36 (4H, m), 8.12 (1H, d), 7.94 (3H, d), 7.86 (1H, s), 7.75~7.81 (3H, m), 7.31~7.62 (19H, m), 7.16 (2H, t) |
| 29 | δ=8.55 (1H, d), 8.36 (4H, m), 8.23 (1H, s), 7.94 (2H, d), 7.75~7.86 (4H, m), 7.29~7.50 (14H, m), 7.16 (1H, t), 6.64 (1H, m), 5.80 (2H, m), 3.62 (1H, t), 2.55 (1H, t), 1.35 (6H, s) |
| 31 | δ=8.55 (1H, d), 8.45 (1H, d), 8.36 (4H, m), 8.05 (1H, d), 93~7.94 (3H, m), 7.86 (1H, s), 7.75~7.81 (3H, m), 7.31~7.56 (16H, m), 7.16 (1H, t) |
| 33 | δ=8.55 (1H, d), 8.45 (1H, d), 8.36 (4H, m), 8.17~8.24 (3H, m), 7.75~7.99 (10H, m), 7.31~7.56 (14H, m), 7.16 (1H, t) |
| 38 | δ=8.55 (1H, d), 8.19 (1H, d), 7.94~7.96 (6H, m), 7.75~7.86 (8H, m), 7.16~7.58 (20H, m) |
| 44 | δ=8.55 (1H, d), 8.45 (1H, d), 8.36 (2H, m), 7.93~7.96 (5H, m), 7.75~7.86 (8H, m), 7.25~7.56 (16H, m), 7.16 (1H, t) |
| 46 | δ=8.55 (1H, d), 8.36~8.38 (5H, m), 8.19 (1H, d), 7.94 (3H, m), 7.86(1H, s), 7.73~7.81 (4H, m), 7.31~7.62 (22H, m), 7.16~7.20 (2H, m) |
| 48 | δ=8.55 (1H, d), 8.45 (1H, d), 8.36 (4H, m), 8.12 (2H, m), 7.68~7.99 (10H, m), 7.31~7.56 (15H, m), 7.16 (1H, t) |
| 54 | δ=8.55 (1H, d), 8.36 (4H, m), 7.75~8.03 (13H, m), 7.31~7.54 (15H, m), 7.16 (1H, t) |
| 58 | δ=8.55 (1H, d), 8.36 (4H, m), 8.21~8.24 (2H, m), 7.74~7.94 (8H, m), 7.31~7.57 (14H, m), 7.16 (1H, t), 1.69 (6H, s) |
| 64 | δ=8.55 (1H, d), 8.30~8.31 (3H, m), 8.13 (1H, d), 7.74~7.94 (16H, m), 7.35~7.58 (13H, m), 7.16 (1H, t) |
| 66 | δ=8.55 (1H, d), 8.29 (2H, m), 8.18~8.19 (2H, m), 7.31~7.99 (24H, m), 7.15~7.20 (3H, m) |
| 68 | δ=8.55 (1H, d), 8.45 (1H, d), 8.29 (2H, d), 8.18 (1H, d), 7.31~7.99 (23H, m), 7.15~7.16 (2H, m) |
| 71 | δ=8.55 (1H, d), 8.36 (2H, m), 8.21~8.24 (2H, m), 7.74~7.96 (12H, m), 7.25~7.57 (16H, m), 7.16 (1H, t), 1.69 (6H, s) |
| 73 | δ=8.55 (1H, d), 8.36 (4H, m), 8.19~8.21 (2H, m), 8.02~8.08 (2H, m), 7.94 (1H, d), 7.86 (1H, s), 7.75~7.68 (4H, m), 7.35~7.60 (15H, m), 7.16~7.20 (2H, m) |
| 83 | δ=8.55~8.56 (2H, m), 8.31~8.38 (2H, m), 7.73~7.94 (13H, m), 7.28~7.49 (17H, m), 7.16 (1H, t) |
| 86 | δ=8.87 (1H, d), 8.55 (1H, d), 8.45 (1H, d), 8.14~8.26 (7H, m), 7.75~7.99 (10H, m), 7.31~7.56 (13H, m), 7.15~7.16 (2H, m) |
| 90 | δ=8.55 (1H, d), 8.45 (1H, d), 8.35 (2H, m), 7.93~7.94 (3H, m), 7.75~7.86 (10H, m), 7.31~7.65 (17H, m), 7.16 (1H, t) |
| 92 | δ=8.39 (1H, d), 8.27~8.30 (2H, m), 8.08~8.18 (3H, m), |
| 96 | δ=8.39 (1H, d), 8.27~8.03 (2H, m), 8.08~8.18 (3H, m), 7.75~7.99 (16H, m), 7.41~7.49 (13H, m), 7.31(1H, d) |
| 100 | δ=8.55~8.56 (2H, m), 8.45 (1H, d), 8.05 (1H, d), 7.73~7.96 (12H, m), 7.25~7.61 (20H, m), 7.16 (1H, t) |
| 101 | δ=8.55 (1H, d), 8.36 (4H, m), 8.19 (1H, d), 7.94~7.98 (2H, m), 7.75~7.86 (4H, m), 7.35~7.58 (13H, m), 7.16~7.25 (3H, m) |
| 103 | δ=8.55 (1H, d), 8.28~8.36 (5H, m), 8.11 (1H, d), 7.94~7.98 (2H, m), 7.69~7.86 (6H, m), 7.35~7.55 (13H, m), 7.25 (1H, d), 7.16 (1H, t) |
| 104 | δ=8.55 (1H, d), 8.36 (4H, m), 7.75~7.99 (11H, m), 7.35~7.54 (14H, m), 7.25 (1H, d), 7.16 (1H, t) |
| 106 | δ=8.55 (1H, d), 8.45 (1H, d), 8.36 (4H, m), 7.93~7.98 (3H, m), 7.75~7.86 (6H, m), 7.41~7.56 (13H, m), 7.25 (1H, d), 7.16 (1H, t) |
| 109 | δ=8.55 (1H, d), 8.36 (4H, m), 7.94~7.98 (3H, m), 7.75~7.86 (5H, m), 7.25~7.54 (16H, m), 7.16 (1H, t) |
| 110 | δ=8.55 (1H, d), 8.36 (4H, m), 7.94~7.98 (3H, m), 7.86 (1H, s), 7.75~7.81 (3H, m), 7.25~7.54 (17H, m), 7.16 (1H, t) |
| 112 | δ=8.55 (1H, d), 8.36 (4H, m), 8.19 (1H, d), 7.94~7.98 (3H, m), 7.73~7.86 (5H, m), 7.35~7.61 (15H, m), 7.16~7.25 (3H, m) |
| 114 | δ=8.55 (1H, d), 8.36 (4H, m), 8.19 (1H, d), 7.94~7.98 (2H, m), 7.86 (1H, s), 7.75~7.81 (3H, m), 7.35~7.62 (20H, m), 7.16~7.25 (3H, m) |
| 116 | δ=8.55 (1H, d), 8.36 (4H, m), 7.94~7.98 (3H, m), 7.86 (1H, s), 7.75~7.81 (3H, m), 7.25~7.54 (17H, m), 7.16 (1H, t) |
| 117 | δ=8.55 (1H, d), 8.36~8.38 (5H, m), 8.19 (1H, d), 7.94~7.98 (3H, m), 7.73~7.86 (5H, m), 7.35~7.61 (14H, m), 7.16~7.25 (3H, m) |
| 120 | δ=8.55 (1H, m), 8.19 (1H, d), 7.94~7.98 (2H, m), 7.75~7.86 (5H, m), 7.16~7.58 (17H, m) |
| 123 | δ=8.55 (1H, d), 8.23 (1H, d), 8.19~8.23 (2H, m), 7.94~7.98 (7H, m), 7.73~7.86 (5H, m), 7.35~7.58 (14H, m), 7.16~7.25 (3H, m) |
| 127 | δ=8.55 (2H, d), 8.36 (4H, m), 8.12 (1H, d), 7.94~7.98 (3H, m), 7.86 (1H, s), 7.75~7.81 (3H, m), 7.35~7.62 (18H, m), 7.25 (1H, d), 7.16 (2H, t) |
| 129 | δ=8.55 (1H, d), 8.36 (4H, m), 8.23 (1H, s), 7.94~7.98 (2H, m), 7.75~7.86 (4H, m), 7.25~7.54 (14H, m), 7.16 (1H, t), 6.64 (1H, m), 5.80 (2H, m), 3.62 (1H, t), 2.55 (1H, t), 1.35 (6H, s) |
| 131 | δ=8.55 (1H, d), 8.45 (1H, d), 8.36 (4H, m), 8.05 (1H, d), 7.93~7.98 (3H, m), 7.75~7.86 (4H, m), 7.33~7.56 (14H, m), 7.25 (1H, d), 7.16 (1H, t) |
| 133 | δ=8.55 (1H, d), 8.45 (1H, d), 8.36 (4H, m), 8.17~8.24 (3H, m), 7.75~7.99 (10H, m), 7.35~7.56 (13H, m), 7.25 (1H, d), 7.16 (1H, t) |
| 135 | δ=9.09 (1H, s), 8.49~8.55 (2H, m), 8.36 (2H, m), 8.16~8.19 (2H, m), 7.94~8.00 (4H, m), 7.75~7.86 (4H, m), 7.35~7.61 (12H, m), 7.16~7.25 (3H, m) |
| 139 | δ=8.55 (1H, d), 8.36~8.38 (3H, m), 7.73~7.99 (15H, m), 7.16 (1H, d), 7.35~7.54 (14H, m), 7.25 (1H, d), 7.16 (1H, t) |
| 142 | δ=8.55~8.56(2H, m), 7.75~7.99 (12H, m), 7.25~7.62 (16H, m), 7.16 (1H, t) |
| 144 | δ=8.55 (1H, d), 8.45 (1H, d), 8.36 (2H, m), 7.93~7.98 (5H, m), 7.75~7.86 (8H, m), 7.35~7.56 (13H, m), 7.25 (3H, d), 7.16 (1H, t) |
| 145 | δ=8.55 (1H, d), 8.36~8.45 (6H, m), 8.05 (1H, d), 7.93~7.98 (4H, m), 7.73~7.86 (5H, m), 7.35~7.61 (15H, m), 7.25 (1H, d), 7.16 (1H, t) |
| 146 | δ=8.55 (1H, d), 8.36~8.38 (5H, m), 8.19 (1H, d), 7.94~7.98 (3H, m), 7.86 (1H, s), 7.73~7.81 (4H, m), 7.40~7.58 (21H, m), 7.16~7.25 (3H, m) |
| 151 | δ=8.55 (1H, m), 8.45 (1H, d), 8.09 (1H, s), 7.93~7.98 (3H, m), 7.75~7.86 (9H, m), 7.25~7.56 (16H, m), 7.16 (1H, t) |
| 156 | δ=8.55 (2H, d), 8.23 (1H, s), 8.12 (1H, d), 7.94~7.98 (7H, m), 7.75~7.86 (4H, m), 7.35~7.62 (17H, m), 7.16~7.25 (4H, m) |
| 157 | δ=8.55 (1H, d), 8.36 (4H, m), 8.24 (1H, d), 7.94~7.98 (3H, m), 7.67~7.86 (6H, m), 7.35~7.57 (13H, m), 7.25 (1H, d), 7.16 (1H, t), 1.69 (6H, s) |
| 160 | δ=8.55~8.56 (2H, m), 8.21~8.24 (2H, m), 7.74~7.98 (15H, m), 7.25~7.57 (13H, m), 7.16 (1H, t), 1.69 (6H, s) |
| 163 | δ=8.55 (1H, d), 8.24~8.30 (4H, m), 8.13 (1H, d), 7.94~7.98 (2H, m), 7.73~7.86 (12H, m), 7.10~7.58 (23H, m) |
| 165 | δ=8.55 (1H, d), 8.13~8.19 (2H, m), 7.94~7.98 (2H, m), 7.75~7.86 (8H, m), 7.35~7.65 (18H, m), 7.16~7.25 (3H, m) |
| 168 | δ=8.55 (1H, d), 8.45 (1H, d), 8.29 (2H, d), 8.18 (1H, d), 7.35~7.99 (22H, m), 7.25 (1H, d), 7.15~7.16 (2H, m) |
| 171 | δ=8.55 (1H, d), 8.36 (2H, m), 8.21~8.24 (2H, m), 7.74~7.98 (12H, m), 7.35~7.57 (13H, m), 7.25 (3H, d), 7.16 (1H, t), 1.69 (6H, s) |
| 176 | δ=8.55 (1H, m), 8.19~8.21 (2H, m), 8.03 (1H, d), 7.94 (1H, d), 7.16~7.86 (29H, m) |
| 177 | δ=8.87 (1H, d), 8.71 (1H, d), 8.55 (1H, d), 8.33 (2H, d), 8.19~8.20 (2H, d), 7.75~7.98 (7H, m), 7.15~7.58 (15H, m) |
| 184 | δ=8.55~8.56 (2H, d), 8.38~8.45 (2H, m), 7.73~8.05 (11H, m), 7.25~7.61 (17H, m), 7.16 (1H, t) |
| 188 | δ=8.55 (1H, d), 8.35 (2H, m), 8.19~8.21 (2H, m), 8.03 (1H, d), 7.94 (1H, d), 7.35~7.86 (28H, m), 7.16~7.20 (2H, m) |
| 190 | δ=8.55 (1H, d), 8.45 (1H, d), 8.35 (2H, m), 7.93~7.98 (3H, m), 7.75~7.86 (10H, m), 7.35~7.65 (16H, m), 7.25 (1H, d), 7.16 (1H, t) |
| 195 | δ=8.30~8.38 (4H, m), 8.13 (1H, d), 7.86~7.98 (6H, m), 7.73~7.81 (14H, m), 7.61 (2H, d), 7.41~7.54 (16H, m), 7.25 (1H, d) |
| 200 | δ=8.55~8.56 (2H, d), 8.45 (1H, d), 7.73~8.05 (13H, m), 7.16~7.61 (21H, m) |
| 201 | δ=8.55 (1H, d), 8.45 (1H, d), 8.36 (4H, m), 8.17~8.24 (4H, m), 7.93~7.98 (3H, m), 7.81~7.86 (2H, m), 7.49~7.56 (11H, m), 7.35 (1H, t), 7.16~7.25 (3H, m) |
| 204 | δ=8.55 (1H, d), 8.45 (1H, d), 8.36 (4H, m), 8.12~8.24 (5H, m), 7.93~8.05 (5H, m), 7.75~7.86 (4H, m), 7.35~7.60 (14H, m), 7.25 (1H, d), 7.16 (1H, t) |
| 205 | δ=8.55 (1H, d), 8.36 (4H, m), 7.75~7.99 (13H, m), 7.25~7.54 (15H, m), 7.16 (1H, t) |
| 210 | δ=8.55 (1H, d), 8.45 (1H, d), 8.36 (4H, m), 8.12 (2H, m), 7.75~7.99 (11H, m), 7.31~7.56 (14H, m), 7.16 (1H, t) |
| 211 | δ=8.55 (1H, d), 8.45 (2H, d), 8.36 (4H, m), 8.17~8.24 (3H, m), 8.05 (1H, d), 7.93~7.94 (4H, m), 7.81~7.86 (2H, m), 7.46~7.60 (12H, m), 7.31~7.35 (2H, m), 7.16 (1H, t) |
| 213 | δ=8.55 (1H, d), 8.36 (4H, m), 7.75~7.99 (13H, m), 7.31~7.50 (15H, m), 7.16 (1H, t) |
| 215 | δ=8.55~8.56 (2H, m), 8.45 (1H, d), 8.31 (1H, d), 8.12 (2H, m), 7.74~7.99 (11H, m), 7.31~7.62 (15H, m), 7.16 (1H, t) |

### <Experimental Example 1>-Manufacture of Organic Light Emitting Device

### 1) Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4'4"-tris[2-naphthyl(phenyl)amino]triphenylamine) and a hole transport layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. The light emitting layer was deposited to 400 Å using a compound described in the following Table 5 as a host and Ir(ppy)₃ (tris (2-phenylpyridine) iridium) as a green phosphorescent dopant by doping the Ir(ppy)₃ to the host by 7%. After that, BCP was deposited to 60 Å as a hole blocking layer, and Alq₃ was deposited to 200 Å thereon as an electron transport layer.

Lastly, an electron injection layer was formed on the electron transport layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 10⁻⁸ torr to 10⁻⁶ torr for each material to be used in the OLED manufacture.

### 2) Driving Voltage and Light Emission Efficiency of Organic Electroluminescent Device

For each of the organic electroluminescent devices manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc.. Properties of the organic electroluminescent devices of the present disclosure are as shown in Table 5.

**[Table 5]**

| | Compo und | Driving Voltage (V) | Efficie ncy (cd/A) | Color Coordinate (x, y) | Lifet ime (T₉₀) |
|---|---|---|---|---|---|
| Comparative Example 1 | A | 5.72 | 45.7 | (0.273, 0.687) | 54 |
| Comparative Example 2 | B | 5.53 | 49.2 | (0.275, 0.674) | 61 |
| Comparative Example 3 | C | 5.33 | 56.5 | (0.270, 0.685) | 46 |
| Comparative Example 4 | D | 5.62 | 47.2 | (0.276, 0.681) | 53 |
| Comparative Example 5 | E | 6.01 | 30.4 | (0.271, 0.684) | 32 |
| Comparative Example 6 | F | 5.65 | 55.1 | (0.280, 0.671) | 50 |
| Comparative Example 7 | G | 5.23 | 56.0 | (0.274, 0.672) | 65 |
| Comparative Example 8 | H | 5.29 | 57.0 | (0.279, 0.673) | 63 |
| Comparative Example 9 | I | 5.70 | 50.5 | (0.281, 0.664) | 55 |
| Comparative Example 10 | J | 5.43 | 48.2 | (0.278, 0.677) | 51 |
| Comparative Example 11 | K | 5.69 | 46.0 | (0.286, 0.681) | 54 |
| Comparative Example 12 | L | 7.02 | 32.4 | (0.273, 0.671) | 30 |
| Example 1 | 1 | 3.70 | 70.8 | (0.278, 0.686) | 135 |
| Example 2 | 4 | 4.12 | 73.5 | (0.276, 0.680) | 126 |
| Example 3 | 5 | 5.20 | 80.5 | (0.271, 0.684) | 147 |
| Example 4 | 6 | 4.55 | 80.4 | (0.279, 0.683) | 150 |
| Example 5 | 7 | 5.12 | 76.1 | (0.274, 0.667) | 136 |
| Example 6 | 9 | 4.09 | 77.0 | (0.287, 0.674) | 144 |
| Example 7 | 10 | 3.74 | 74.2 | (0.283, 0.677) | 130 |
| Example 8 | 14 | 3.89 | 79.6 | (0.279, 0.676) | 149 |
| Example 9 | 16 | 4.14 | 80.3 | (0.272, 0.679) | 135 |
| Example 10 | 22 | 3.98 | 76.7 | (0.273, 0.671) | 133 |
| Example 11 | 25 | 3.77 | 74.4 | (0.271, 0.678) | 147 |
| Example 12 | 27 | 3.86 | 70.9 | (0.279, 0.675) | 146 |
| Example 13 | 29 | 3.74 | 71.6 | (0.287, 0.673) | 144 |
| Example 14 | 31 | 3.88 | 76.5 | (0.271, 0.672) | 141 |
| Example 15 | 33 | 3.89 | 80.4 | (0.288, 0.680) | 150 |
| Example 16 | 38 | 3.73 | 77.6 | (0.286, 0.681) | 143 |
| Example 17 | 44 | 4.86 | 75.6 | (0.274, 0.678) | 131 |
| Example 18 | 46 | 4.90 | 74.9 | (0.274, 0.677) | 129 |
| Example 19 | 48 | 5.17 | 79.9 | (0.278, 0.679) | 147 |
| Example 20 | 54 | 4.63 | 74.5 | (0.270, 0.674) | 127 |
| Example 21 | 58 | 5.17 | 80.4 | (0.271, 0.687) | 143 |
| Example 22 | 64 | 4.69 | 79.2 | (0.284, 0.683) | 144 |
| Example 23 | 66 | 3.80 | 77.1 | (0.283, 0.680) | 141 |
| Example 24 | 68 | 4.76 | 74.2 | (0.279, 0.688) | 131 |
| Example 25 | 71 | 4.85 | 76.8 | (0.285, 0.669) | 139 |
| Example 26 | 73 | 5.14 | 77.5 | (0.281, 0.676) | 134 |
| Example 27 | 83 | 3.89 | 76.1 | (0.277, 0.682) | 148 |
| Example 28 | 86 | 3.79 | 75.3 | (0.288, 0.682) | 130 |
| Example 29 | 90 | 3.88 | 78.0 | (0.274, 0.672) | 125 |
| Example 30 | 92 | 4.15 | 71.8 | (0.276, 0.674) | 127 |
| Example 31 | 96 | 4.53 | 77.6 | (0.275, 0.673) | 126 |
| Example 32 | 100 | 4.73 | 80.4 | (0.279, 0.674) | 136 |
| Example 33 | 101 | 3.60 | 57.6 | (0.278, 0.677) | 148 |
| Example 34 | 103 | 5.10 | 70.4 | (0.280, 0.675) | 174 |
| Example 35 | 104 | 3.69 | 59.5 | (0.273, 0.670) | 169 |
| Example 36 | 106 | 3.89 | 69.7 | (0.270, 0.668) | 175 |
| Example 37 | 109 | 3.61 | 66.5 | (0.271, 0.663) | 166 |
| Example 38 | 110 | 4.84 | 61.7 | (0.274, 0.681) | 163 |
| Example 39 | 112 | 4.44 | 78.5 | (0.272, 0.666) | 171 |
| Example 40 | 114 | 5.06 | 66.5 | (0.273, 0.665) | 173 |
| Example 41 | 116 | 3.82 | 61.1 | (0.271, 0.660) | 159 |
| Example 42 | 117 | 3.94 | 62.8 | (0.263, 0.671) | 168 |
| Example 43 | 123 | 4.09 | 68.5 | (0.280, 0.683) | 166 |
| Example 44 | 127 | 4.18 | 63.8 | (0.277, 0.679) | 167 |
| Example 45 | 129 | 5.04 | 69.4 | (0.278, 0.679) | 159 |
| Example 46 | 131 | 3.84 | 58.8 | (0.274, 0.680) | 174 |
| Example 47 | 133 | 4.06 | 57.6 | (0.279, 0.677) | 166 |
| Example 48 | 135 | 3.69 | 62.2 | (0.280, 0.686) | 167 |
| Example 49 | 139 | 4.14 | 63.8 | (0.283, 0.681) | 169 |
| Example 50 | 142 | 4.48 | 67.0 | (0.287, 0.685) | 170 |
| Example 51 | 144 | 4.02 | 67.2 | (0.284, 0.681) | 173 |
| Example 52 | 145 | 4.53 | 69.8 | (0.285, 0.680) | 155 |
| Example 53 | 146 | 4.87 | 64.8 | (0.284, 0.677) | 159 |
| Example 54 | 151 | 4.94 | 64.0 | (0.277, 0.680) | 169 |
| Example 55 | 156 | 3.86 | 66.7 | (0.278, 0.673) | 167 |
| Example 56 | 157 | 3.74 | 69.2 | (0.280, 0.672) | 166 |
| Example 57 | 160 | 3.86 | 64.0 | (0.279, 0.675) | 173 |
| Example 58 | 163 | 3.78 | 67.4 | (0.289, 0.674) | 170 |
| Example 59 | 165 | 3.71 | 68.5 | (0.271, 0.675) | 177 |
| Example 60 | 168 | 3.64 | 66.8 | (0.270, 0.672) | 168 |
| Example 61 | 171 | 5.05 | 67.5 | (0.277, 0.671) | 164 |
| Example 62 | 176 | 4.31 | 68.7 | (0.273, 0.677) | 154 |
| Example 63 | 177 | 4.49 | 68.7 | (0.274, 0.674) | 159 |
| Example 64 | 184 | 4.07 | 69.0 | (0.270, 0.671) | 168 |
| Example 65 | 188 | 4.18 | 57.9 | (0.280, 0.670) | 173 |
| Example 66 | 190 | 4.48 | 59.4 | (0.289, 0.669) | 151 |
| Example 67 | 195 | 4.69 | 58.1 | (0.279, 0.670) | 168 |
| Example 68 | 200 | 5.08 | 59.2 | (0.289, 0.667) | 157 |
| Example 69 | 210 | 5.14 | 70.5 | (0.278, 0.674) | 131 |
| Example 70 | 211 | 4.97 | 75.9 | (0.275, 0.673) | 139 |
| Example 71 | 213 | 3.85 | 74.4 | (0.274, 0.687) | 141 |
| Example 72 | 215 | 3.60 | 78.6 | (0.281, 0.683) | 146 |

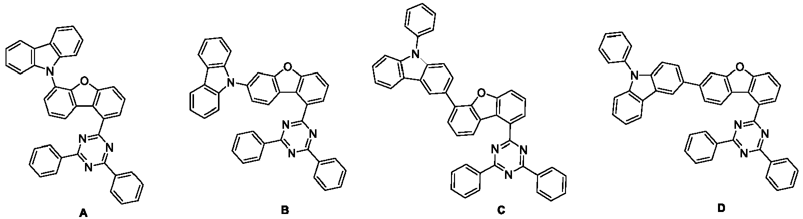

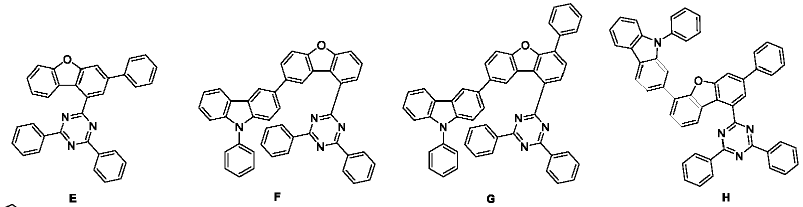

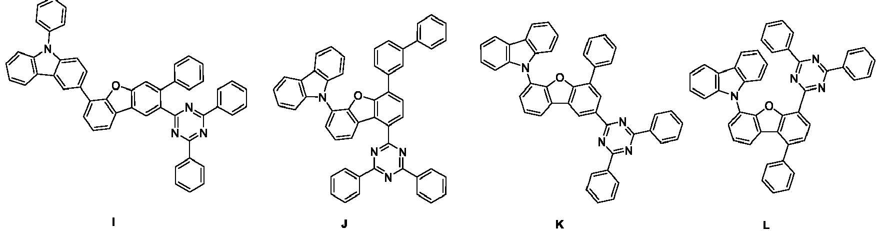

As seen from Table 5, it was identified that, by the heterocyclic compound according to the present application having a specific substituent at No. 1 and No. 3 positions of one side benzene ring of the dibenzofuran and having a carbazole-based substituent on the other side benzene ring, an organic light emitting device comprising the same had properties of excellent lifetime and efficiency.

The compound according to the present application is a tri-substituted compound in which the carbazole-based substituent substituting one side benzene ring of the dibenzofuran is a substituent having hole transport characteristics (hole transport character moiety), N-Het substituting a No. 1 position of the other side benzene ring of the dibenzofuran is a substituent having electron transport characteristics (electron transport character moiety), and in addition, an Ar substituent is included at the No. 3 position. In other words, it was identified that, when Ar was not substituted, the No. 3 position of the dibenzofuran was relatively electron deficient, and by forming an Ar substituent such as Chemical Formula 1 of the present application at a No. 3 position of the dibenzofuran, electrons became abundant increasing structural stability, and as a result, the organic light emitting device comprising the same had properties of enhancing lifetime and current flow.

As seen from Table 5, Comparative Examples 1 to 6 are cases comprising a heterocyclic compound in which at least one of carbazole-based substituent/Ar/N-Het is not provided in the dibenzofuran, and it was identified that the molecular weight herein was smaller than the molecular weight of the compound having three types of substituents bonding to the dibenzofuran leading to a smaller Td (95%) value, and as a result, the lifetime was not favorable due to relatively decreased thermal stability.

Particularly, oxygen of the dibenzofuran has greater electronegativity compared to carbon, and tends to attract electrons of adjacent carbons, and due to such properties, a No. 4 position of the dibenzofuran is relatively electron deficient compared to other positions. Accordingly, it was identified that a much more stable compound in terms of material structure was obtained when bonding the carbazole-based substituent to the No. 4 position, a relatively electron deficient position, compared to when bonding to the No. 1 or No. 2 position of the dibenzofuran.

In addition, Chemical Formula 1 of the present application has N-Het substituting a No. 1 position of the benzene ring. Dibenzofuran has different electron density by each position due to resonance, and the No. 1 position of the dibenzofuran is positively charged compared to the No. 2 and No. 4 positions due to resonance. Accordingly, it was identified that a more stable compound in terms of material structure was obtained when boding N-Het having an electron transport ability to the No. 1 position that is relatively positively charged in the dibenzofuran.

In addition, the N-carbazole substituting the dibenzofuran as in Chemical Formula 1 of the present application is capable of directly transferring lone pair electrons that the nitrogen has to the dibenzofuran core due to resonance. Accordingly, it was identified that an excellent electron transport ability was obtained when having an N-carbazole substituent compared to when having a carbazole substituent substituted with a phenyl group (linking to carbon of carbazole) (Comparative Examples 4 and 6 to 9).

Thermal dissociation temperature (T_{d}) values for the compounds were compared and shown in the following Table 6. The Td (95%) values measured using a thermogravimetric analyzer are as shown in the following Table 6.

**[Table 6]**

| | Compound | T_{d} (95%) (°C) |
|---|---|---|
| Comparative Example 1-1 | A1 | 420 |
| Comparative Example 2-1 | B1 | 427 |
| Example 1 | 1 | 450 |
| Example 2 | 4 | 478 |
| Example 3 | 5 | 459 |
| Example 4 | 6 | 475 |
| Example 5 | 7 | 461 |
| Example 6 | 9 | 470 |
| Example 7 | 10 | 475 |
| Example 8 | 14 | 481 |
| Example 9 | 16 | 445 |
| Example 10 | 22 | 465 |
| Example 11 | 25 | 473 |
| Example 12 | 27 | 462 |
| Example 13 | 29 | 463 |
| Example 14 | 31 | 453 |
| Example 15 | 33 | 473 |
| Example 16 | 38 | 470 |
| Example 17 | 44 | 488 |
| Example 18 | 46 | 459 |
| Example 19 | 48 | 476 |
| Example 20 | 54 | 498 |
| Example 21 | 58 | 449 |
| Example 22 | 64 | 471 |
| Example 23 | 66 | 465 |
| Example 24 | 68 | 473 |
| Example 25 | 71 | 449 |
| Example 26 | 73 | 481 |
| Example 27 | 83 | 475 |
| Example 28 | 86 | 480 |
| Example 29 | 90 | 476 |
| Example 30 | 92 | 452 |
| Example 31 | 96 | 463 |
| Example 32 | 100 | 452 |
| Example 33 | 101 | 455 |
| Example 34 | 103 | 459 |
| Example 35 | 104 | 463 |
| Example 36 | 106 | 477 |
| Example 37 | 109 | 453 |
| Example 38 | 110 | 472 |
| Example 39 | 112 | 458 |
| Example 40 | 114 | 487 |
| Example 41 | 116 | 473 |
| Example 42 | 117 | 477 |
| Example 43 | 123 | 450 |
| Example 44 | 127 | 486 |
| Example 45 | 129 | 472 |
| Example 46 | 131 | 481 |
| Example 47 | 133 | 483 |
| Example 48 | 135 | 466 |
| Example 49 | 139 | 476 |
| Example 50 | 142 | 455 |
| Example 51 | 144 | 469 |
| Example 52 | 145 | 487 |
| Example 53 | 146 | 466 |
| Example 54 | 151 | 479 |
| Example 55 | 156 | 455 |
| Example 56 | 157 | 473 |
| Example 57 | 160 | 484 |
| Example 58 | 163 | 477 |
| Example 59 | 165 | 463 |
| Example 60 | 168 | 458 |
| Example 61 | 171 | 472 |
| Example 62 | 176 | 466 |
| Example 63 | 177 | 450 |
| Example 64 | 184 | 475 |
| Example 65 | 188 | 449 |
| Example 66 | 190 | 487 |
| Example 67 | 195 | 455 |
| Example 68 | 200 | 453 |
| Example 69 | 210 | 467 |
| Example 70 | 211 | 462 |
| Example 71 | 213 | 473 |
| Example 72 | 215 | 471 |
| Example 1-1 | C1 | 435 |
| Example 2-1 | D1 | 437 |
| Example 3-1 | E1 | 440 |
| Example 4-1 | F1 | 438 |
| Example 5-1 | G1 | 433 |
| Example 6-1 | H1 | 449 |

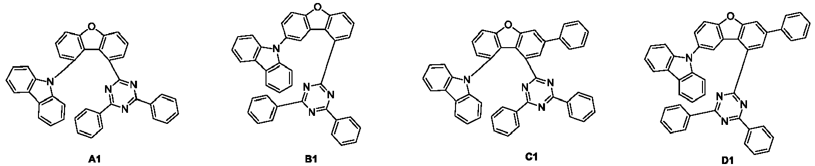

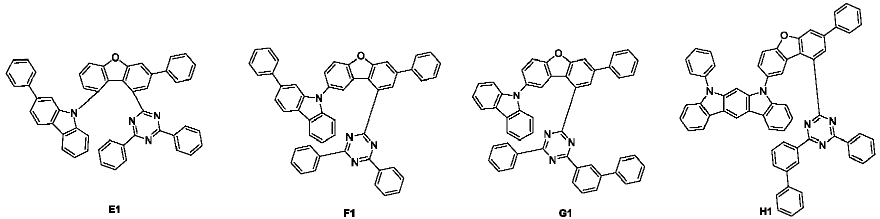

As seen from Table 6, it was identified that the heterocyclic compound of Chemical Formula 1 according to the present application had a higher Td value by approximately 40°C compared to the compounds of Comparative Example 1-1 and Comparative Example 2-1. This indicates that the tri-substituted compound in which the carbazole-based substituent substituting one side benzene ring of the dibenzofuran is a substituent having hole transport characteristics (hole transport character moiety), N-Het substituting a No. 1 position of the other side benzene ring of the dibenzofuran is a substituent having electron transport characteristics (electron transport character moiety), and in addition, an Ar substituent is included at the No. 3 position has particularly superior thermal stability compared to the disubstituted compounds (Comparative Example 1-1 and Comparative Example 2-1).

Particularly, Examples 1 to 72 of Table 6 are cases corresponding to Chemical Formulae 2 and 3 according to the present application, and tend to have a T_{d} (95%) value higher by approximately 10°C to 40°C compared to other cases (Examples 1-1 to 6-1), and properties of particularly superior thermal stability is obtained. In other words, it was identified that, as the No. 3 or No. 4 position is substituted with a carbazole-based substituent and two substituents were formed at No. 1 and No. 3 positions of the opposite side benzene ring in Chemical Formulae 2 and 3, the molecular weight increased, and stability of the structure itself increased.

## Claims

1. A heterocyclic compound represented by the following Chemical Formula 1: 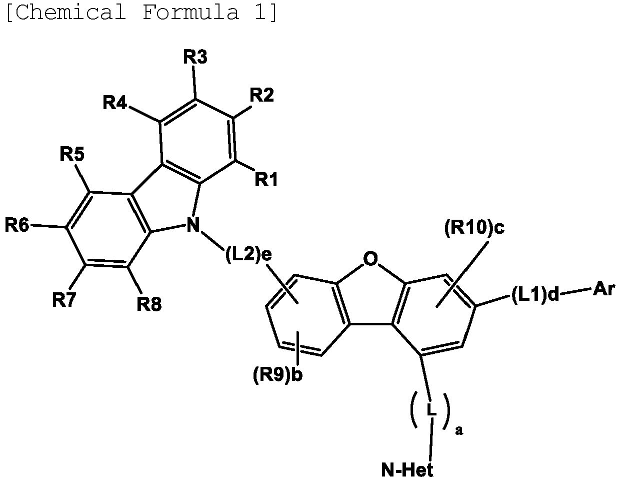 wherein, in Chemical Formula 1,
N-Het is a monocyclic or polycyclic heterocyclic group substituted or unsubstituted, and comprising one or more Ns;
R1 to R10 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R" and -NRR', or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring; L, L1 and L2 are the same as or different from each other, and each independently a direct bond; a substituted or unsubstituted C6 to C60 arylene group; or a substituted or unsubstituted C2 to C60 heteroarylene group;
Ar is a substituted or unsubstituted C1 to C60 alkyl group; a substituted or unsubstituted C6 to C60 aryl group; a substituted or unsubstituted C2 to C60 heteroaryl group; - P(=O)RR'; or -SiRR'R";
R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group;
a, d and e are the same as or different from each other, and each an integer of 0 to 4;
b is an integer of 0 to 3;
c is an integer of 0 to 2; and
when a to e are 2 or greater, substituents in the parentheses are the same as or different from each other.

2. The heterocyclic compound of Claim 1, wherein Chemical Formula 1 is represented by any one of the following Chemical Formulae 2 to 5: 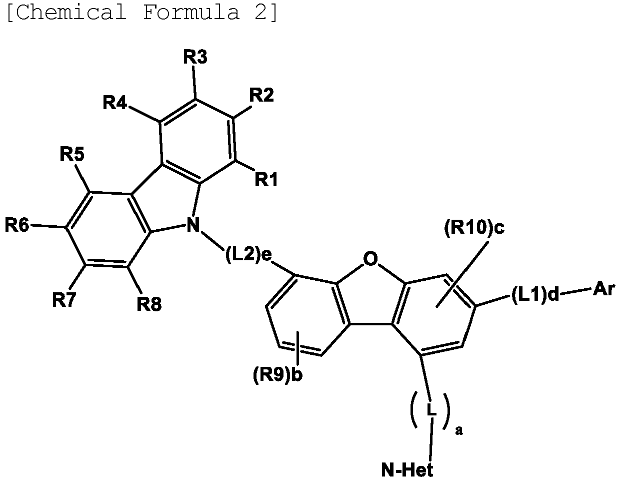 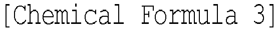 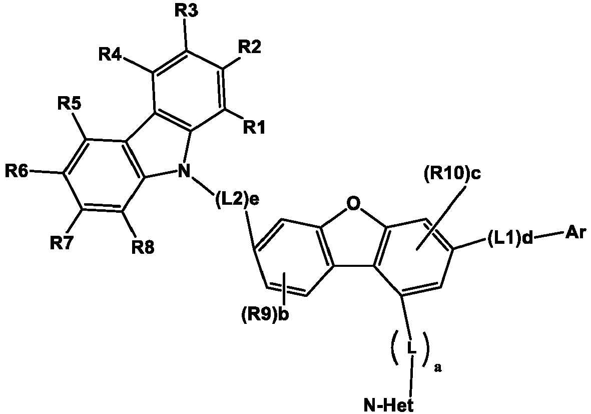 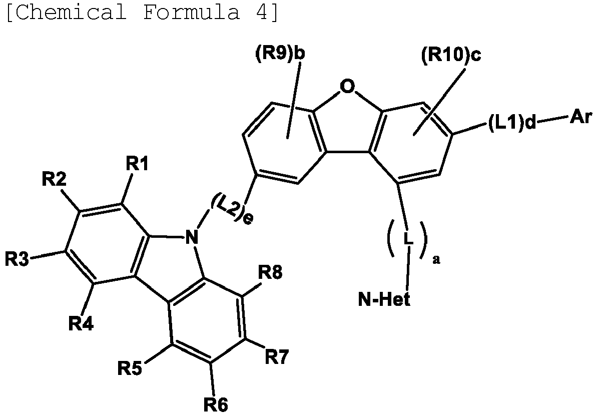 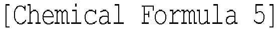 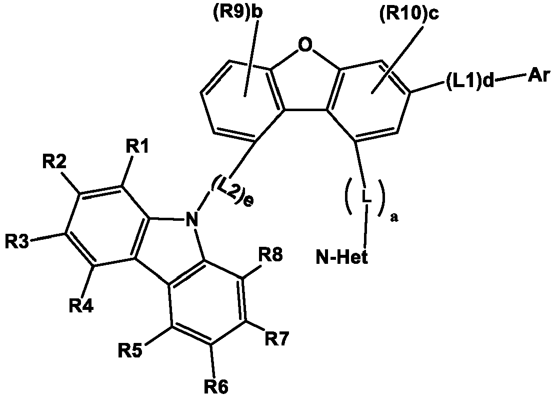 in Chemical Formulae 2 to 5,
R1 to R10, N-Het, Ar, L, L1, L2, a, b, c, d and e have the same definitions as in Chemical Formula 1.

3. The heterocyclic compound of Claim 1, wherein Chemical Formula 1 is represented by the following Chemical Formula 6 or Chemical Formula 7: 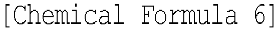 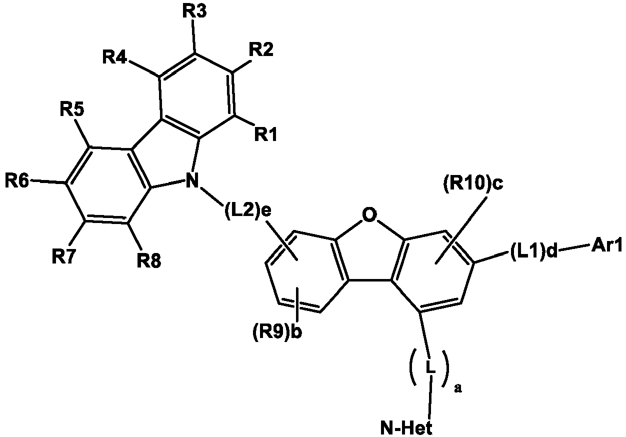 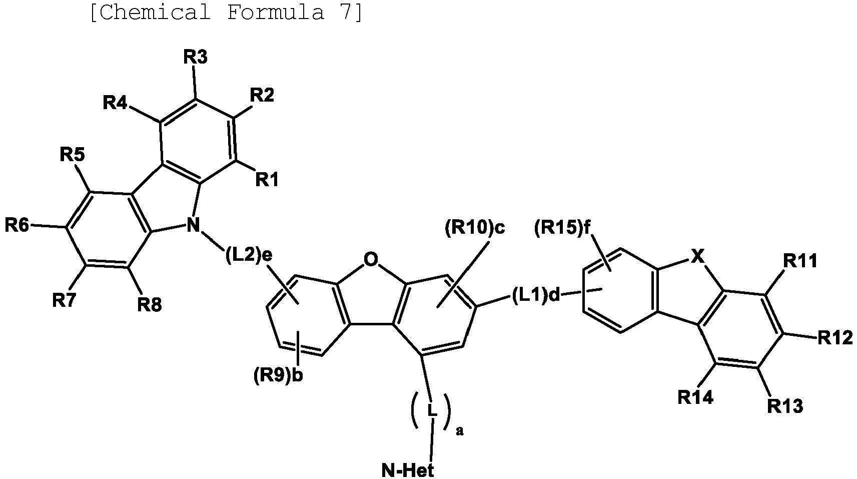 in Chemical Formulae 6 and 7,
R1 to R10, N-Het, L, L1, L2, a, b, c, d and e have the same definitions as in Chemical Formula 1;
Ar1 is a substituted or unsubstituted C6 to C60 aryl group; X is O or S;
R11 to R15 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R" and -NRR', or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring;
R, R' and R" have the same definitions as in Chemical Formula 1; and
f is an integer of 0 to 3, and when f is 2 or greater, substituents in the parentheses are the same as or different from each other.

4. The heterocyclic compound of Claim 1, wherein N-Het of Chemical Formula 1 is a substituted or unsubstituted a triazine group; a substituted or unsubstituted pyrimidine group; a substituted or unsubstituted pyridine group; a substituted or unsubstituted quinoline group; a substituted or unsubstituted 1,10-phenanthroline group; a substituted or unsubstituted 1,7-phenanthroline group; a substituted or unsubstituted quinazoline group; or a substituted or unsubstituted benzimidazole group.

5. The heterocyclic compound of Claim 1, wherein R9 and R10 are hydrogen.

6. The heterocyclic compound of Claim 1, wherein Chemical Formula 1 is represented by any one of the following compounds:

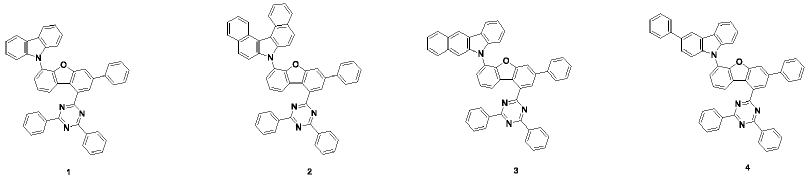

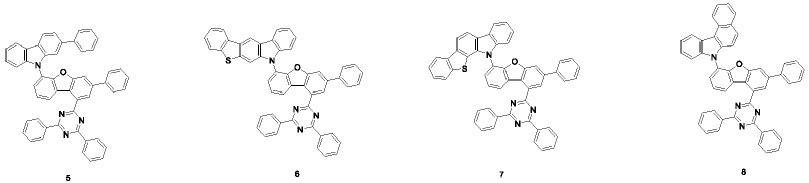

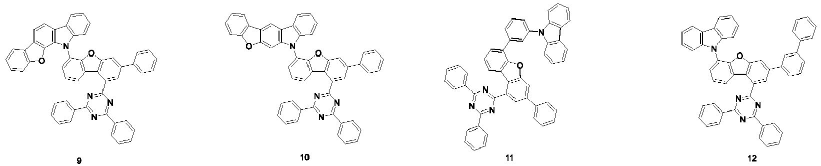

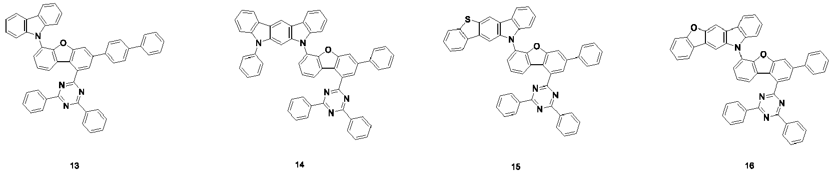

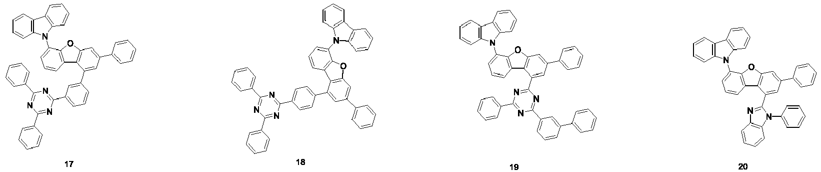

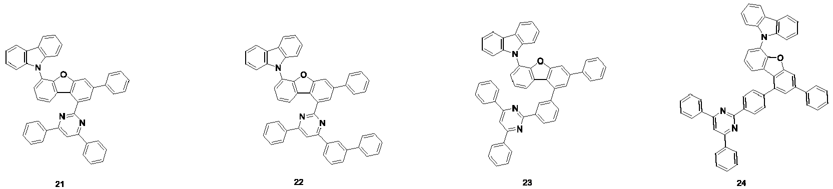

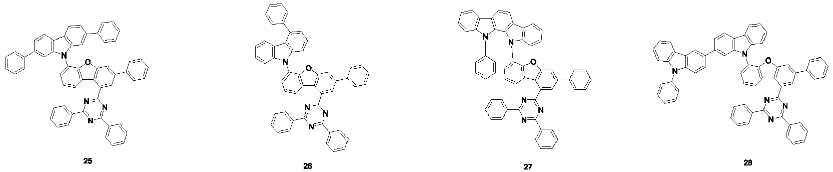

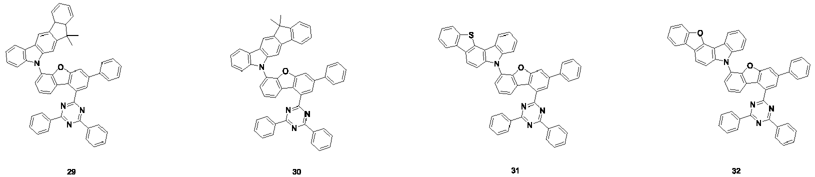

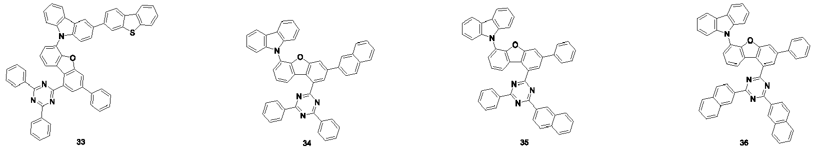

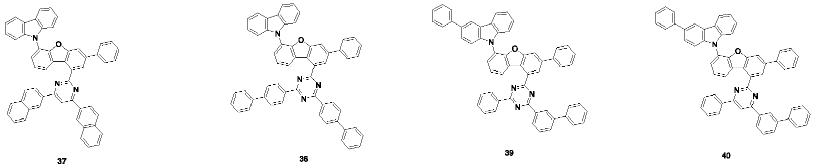

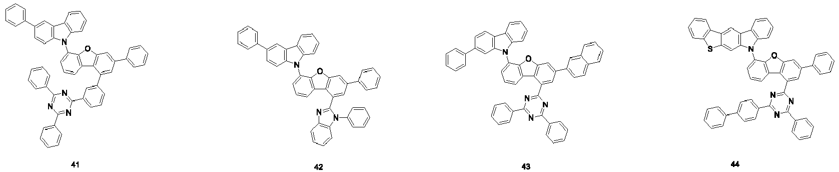

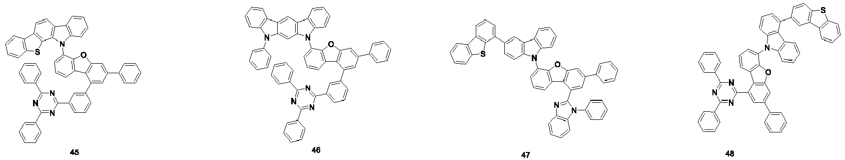

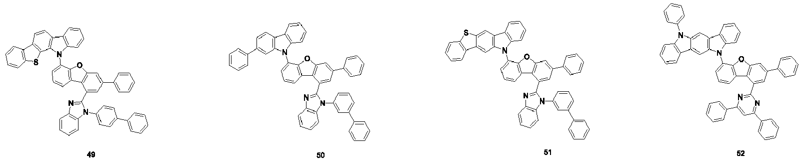

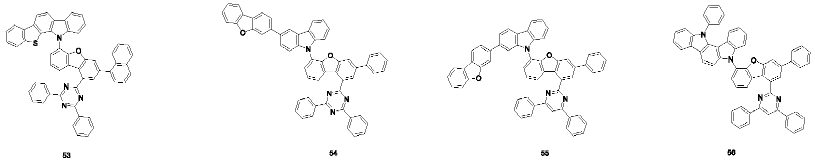

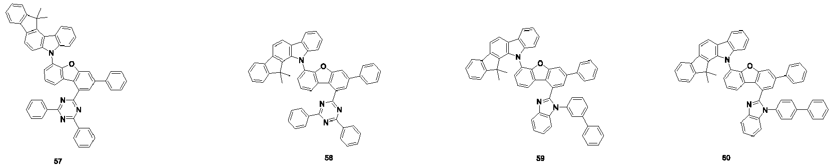

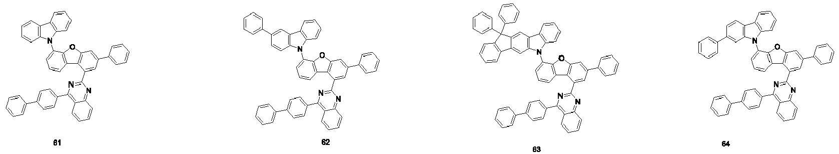

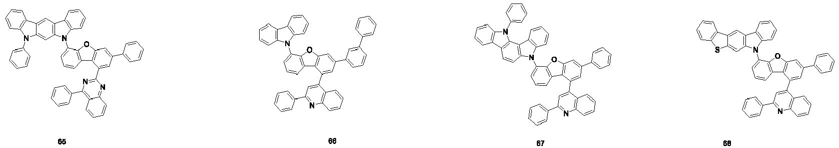

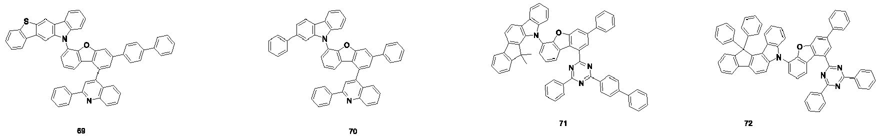

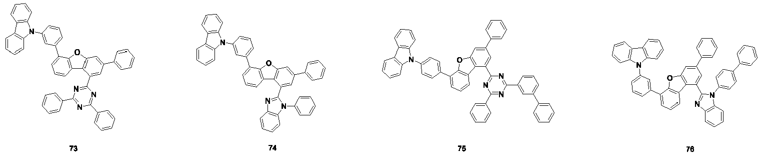

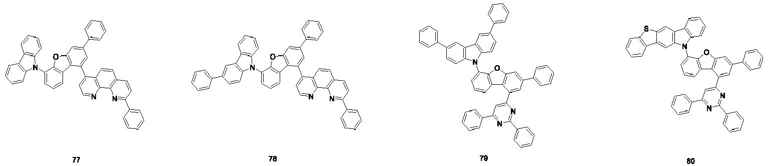

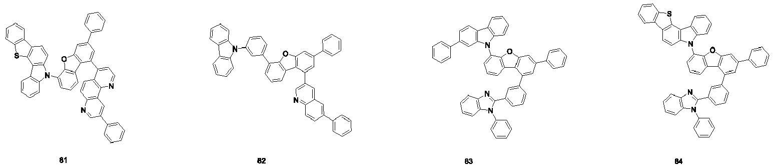

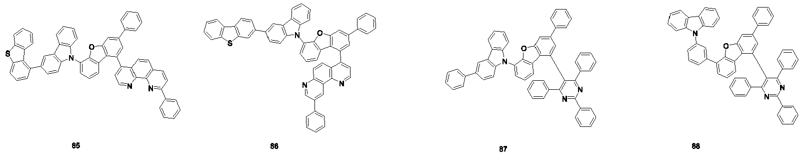

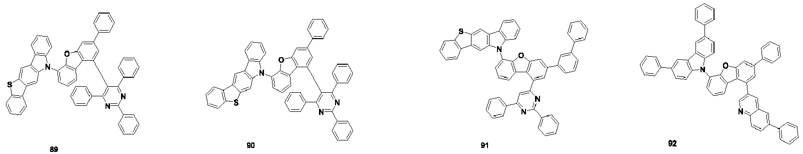

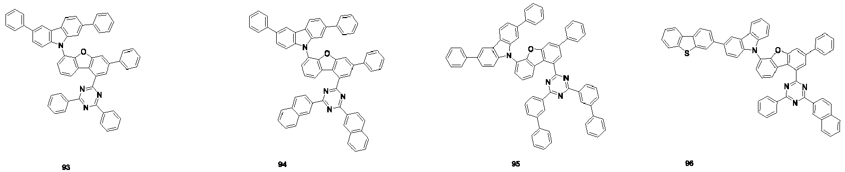

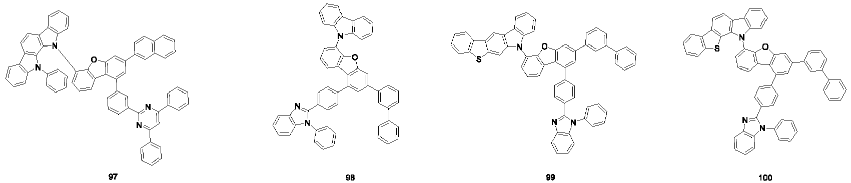

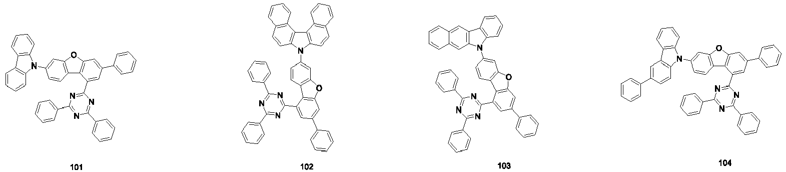

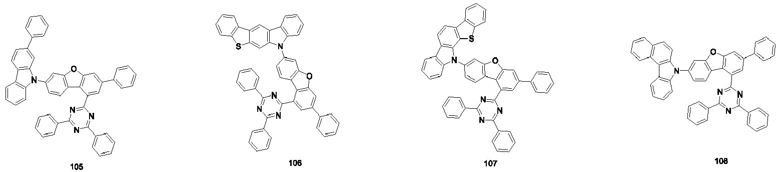

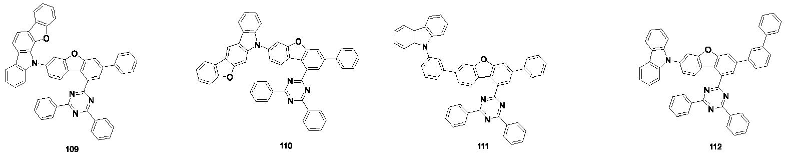

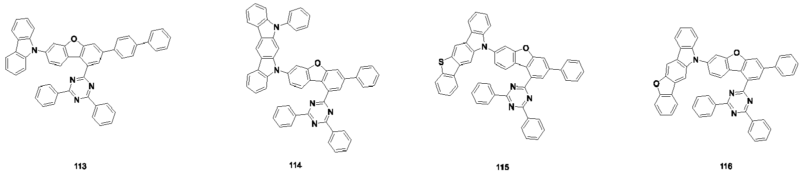

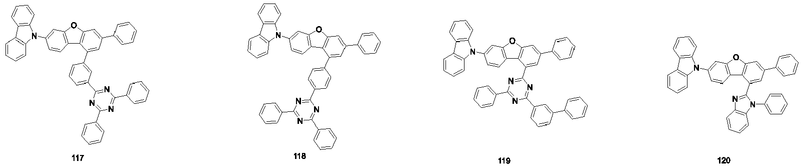

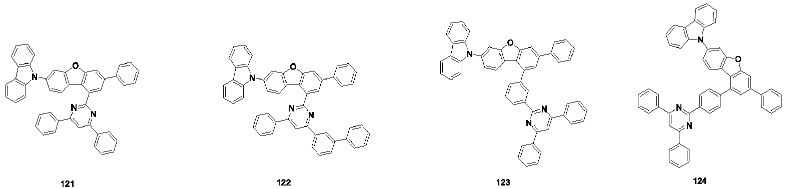

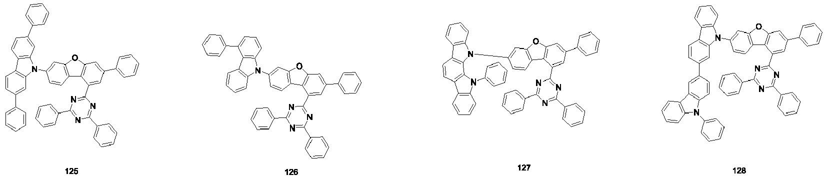

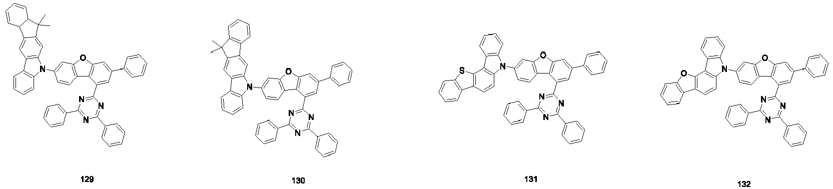

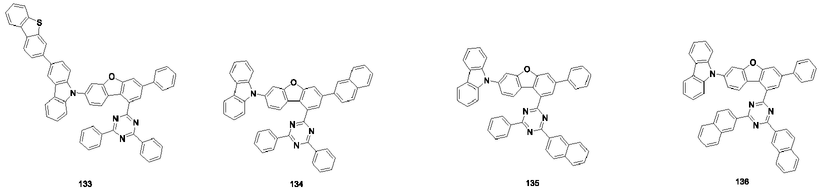

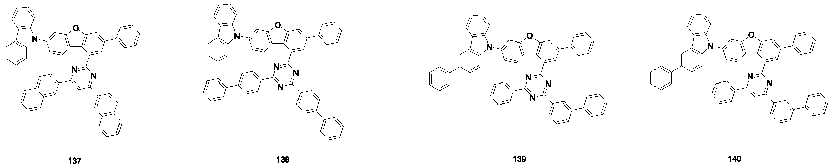

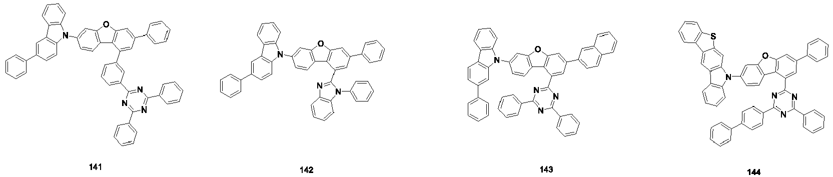

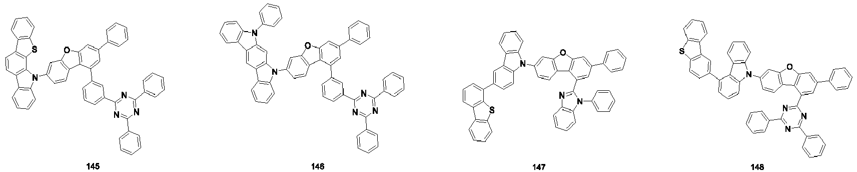

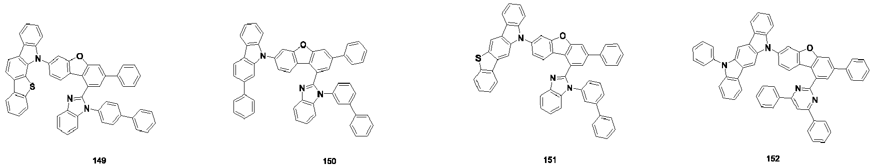

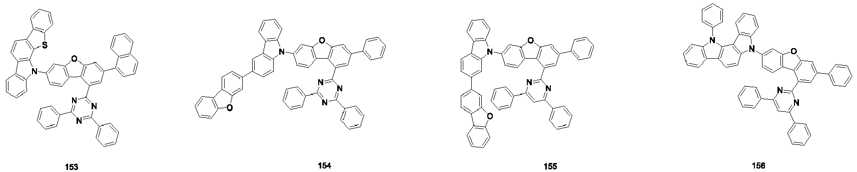

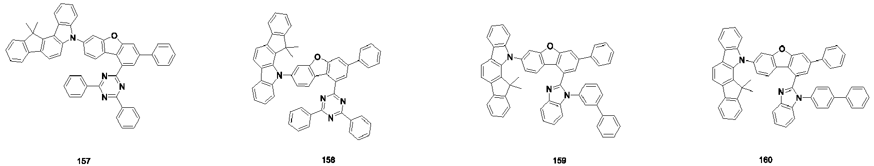

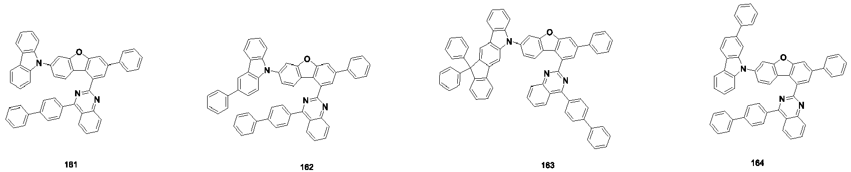

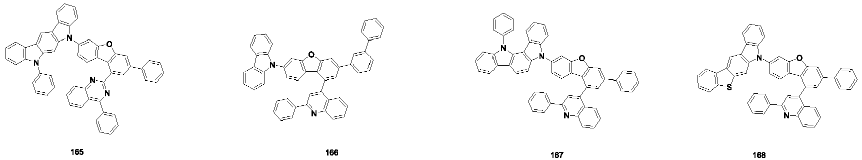

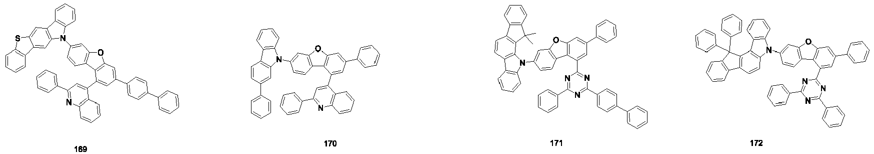

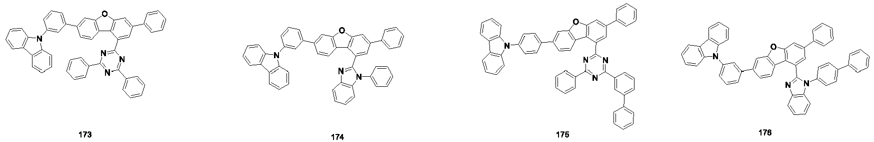

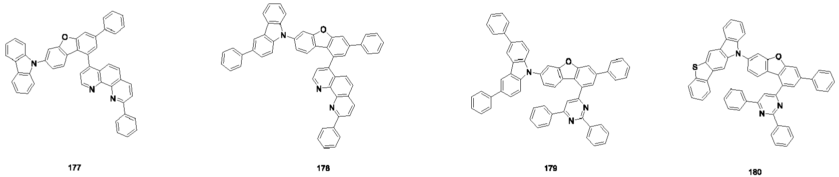

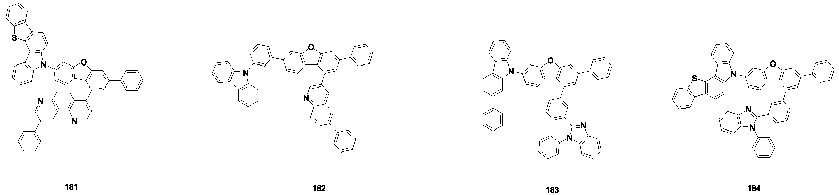

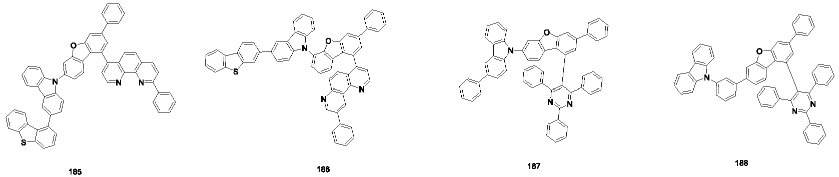

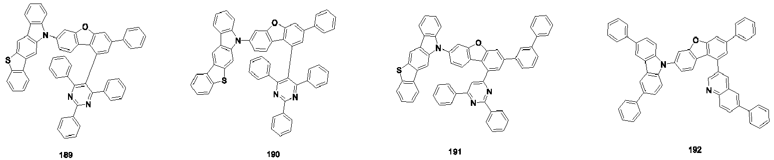

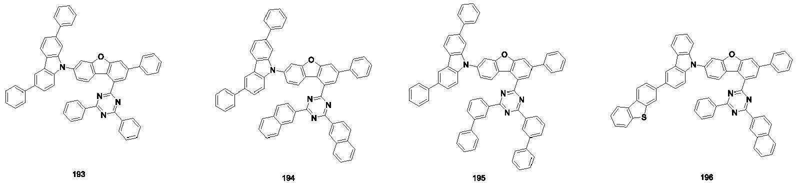

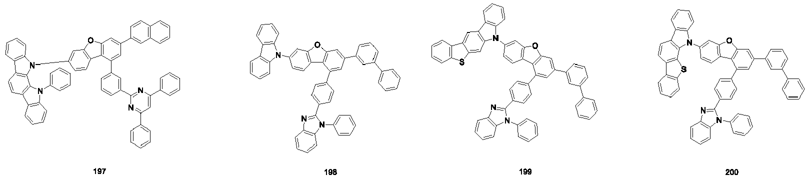

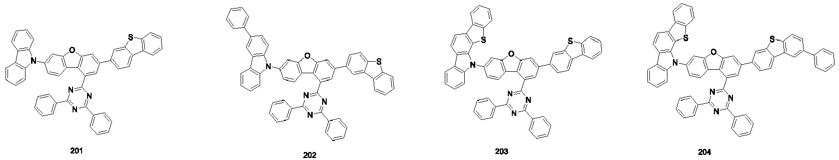

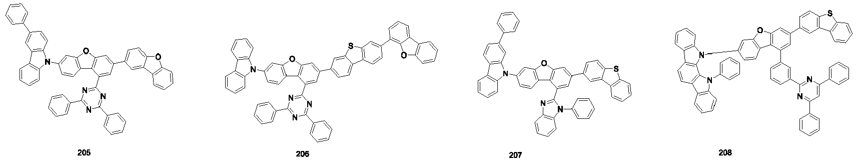

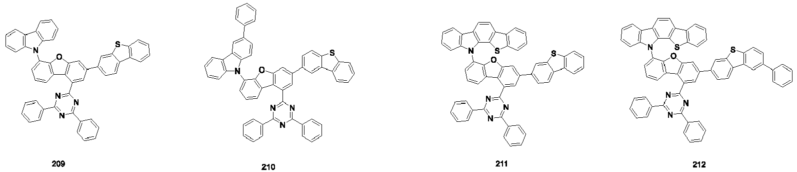

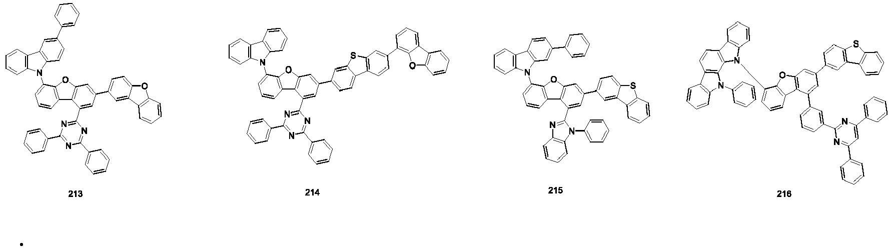

7. An organic light emitting device comprising:
a first electrode;
a second electrode provided opposite to the first electrode; and
one or more organic material layers provided between the first electrode and the second electrode,
wherein one or more layers of the organic material layers comprise the heterocyclic compound of any one of Claims 1 to 6.

8. The organic light emitting device of Claim 7, wherein the organic material layer comprises a light emitting layer, and the light emitting layer comprises the heterocyclic compound.

9. The organic light emitting device of Claim 7, wherein the organic material layer comprises a light emitting layer, the light emitting layer comprises a host material, and the host material comprises the heterocyclic compound.

10. The organic light emitting device of Claim 7, wherein the organic material layer comprises an electron injection layer or an electron transport layer, and the electron transport layer or the electron injection layer comprises the heterocyclic compound.

11. The organic light emitting device of Claim 7, wherein the organic material layer comprises an electron blocking layer or a hole blocking layer, and the electron blocking layer or the hole blocking layer comprises the heterocyclic compound.

12. The organic light emitting device of Claim 7, further comprising one, two or more layers selected from the group consisting of a light emitting layer, a hole injection layer, a hole transport layer, an electron injection layer, an electron transport layer, an electron blocking layer and a hole blocking layer.
